(19) Europäisches Patentamt
European Patent Office
Office européen des brevets

(11) Veröffentlichungsnummer: **0 085 944**
**B1**

(12) # EUROPÄISCHE PATENTSCHRIFT

(45) Veröffentlichungstag der Patentschrift: 09.04.86

(51) Int. Cl.⁴: C 09 J 3/00, A 61 L 15/06

(21) Anmeldenummer: 83100986.5

(22) Anmeldetag: 03.02.83

(54) **Chirurgische Bindemittelsysteme zum Verkleben von körpereigenem Hartgewebe gewünschtenfalls mit Kunststoff und/oder Metall.**

(30) Priorität: 10.02.82 DE 3204504
09.08.82 DE 3229635

(43) Veröffentlichungstag der Anmeldung: 17.08.83 Patentblatt 83/33

(45) Bekanntmachung des Hinweises auf die Patenterteilung: 09.04.86 Patentblatt 86/15

(84) Benannte Vertragsstaaten: AT BE CH DE FR GB IT LI NL SE

(56) Entgegenhaltungen: Keine Entgegenhaltungen

(73) Patentinhaber: Henkel Kommanditgesellschaft auf Aktien, Postfach 1100 Henkelstrasse 67, D-4000 Düsseldorf-Holthausen (DE)

(72) Erfinder: Ritter, Wolfgang, Dr., Hochdahler Strasse 22, D-4010 Hilden (DE)

EP 0 085 944 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft neue, unter dem Einfluß von Luftsauerstoff selbsthärtende Kunststoffsysteme, die als chirurgische Binde- bzw. Klebemittel für das Verkleben von körpereigenem Hartgewebe und/oder zur in-situ-Bildung von Kunststoff-Formteilen im Zuge chirurgischer Arbeiten in der Human- und Tiermedizin Verwendung finden können. Eine besondere Eigenart der mittels der Erfindung hergestellten neuen Kunststoffe liegt in ihrer Resorbierbarkeit durch körpereigene Abbaureaktionen, wobei gewünschtenfalls auch Einfluß auf die Zeitdauer des Resorptionsprozesses genommen werden kann.

**Zum Stand der Technik**

Die feste, dauerhafte Vereinigung von getrennten Knochenteilen oder die Fixierung von künstlichen Implantaten im bzw. am Knochen- oder Zahnmaterial ist eine alte Wunschvorstellung der Chirurgen. Bisher sind vorwiegend Polymethacrylate, Epoxidharz- und Polyurethansysteme untersucht worden. In der Praxis haben sich bisher ausschließlich die Polymethacrylate durchgesetzt. Sie sind vorwiegend geeignet für Implantation von Gelenkprothesen, die Zement-Metall-Osteosynthese, Wirbelsäulenfusionen und Defektabdeckungen am Schädeldach bzw. Implantation von Stützmaterial in der Gesichtschirurgie.

Neben diesen Anwendungen besteht der Wunsch zusätzliche Anwendungsfelder für Knochenklebstoffe bzw. Knochenzemente zu erschließen, beispielsweise im Zusammenhang mit einer anatomischen Reposition, Fixierung und Retention von Knochenfragmenten bei Trümmer- oder Gelenkbrüchen. Angestrebtes Ziel ist insbesondere eine dauerhafte Vereinigung von Knochenfragmenten mit dem Ziel einer sofortigen Belastbarkeit nach der Aushärtung des Zementes bzw. Klebstoffes. Besonders wünschenswert erscheint die reversible Reposition, Fixierung und Retention von Knochenfragmenten mit Hilfe resorbierbarer Klebstoffsysteme. Hier soll der Knochenklebstoff bzw. -Zement synchron zur Knochenneubildung vom Körper resorbiert werden. Weiterhin ist von Bedeutung die reversible Stabilisierung von Frakturen durch Platten oder Schienen, die mittels resorbierbarer Klebstoffe befestigt werden. Hier kann bei der Verwendung von Schienen oder Platten aus Metall bzw. Kunststoff auf das Anbringen von Bohrlöchern im Knochen und die Verwendung von Schrauben verzichtet werden. Bei Verwendung resorbierbarer Stützmaterialien z.B. aus Polymilchsäure oder Polyglycolsäure könnte zusätzlich die zweite Operation entfallen.

An Klebstoffe bzw. Zemente für solche Zwecke sind u.a. die folgenden Forderungen zu stellen: Chemische Stabilität, Erhaltung der physikalischen Eigenschaften auch unter Einwirkung von Körperflüssigkeit, innerhalb des benötigten zeitlichen Rahmens, biologische Verträglichkeit, keine Kanzerogenität, keine Allergieauslösung oder sonstige Überempfindlichkeit, volle mechanische Belastbarkeit nach Aushärtung, in-situ-Herstellbarkeit von Kunststoffteilen in der gewünschten Form, gute Sterilisierbarkeit, angemessene Abbindezeit, möglichst geringe Volumenänderung sowie Wärmeentwicklung beim Abbinden. Speziell für das Gebiet resorbierbarer Kunststoffmaterialien gelten die Zusatzforderungen der Resorbierbarkeit im vorgegebenen zeitlichen Rahmen durch körpereigene Abbauvorgänge sowie Freiheit von schädlichen Folgereaktionen durch Abbauprodukte.

Die bisher ausschließlich eingesetzten Methacrylatsysteme bestehen aus den folgenden Komponenten:
einem oder mehreren radikalisch polymerisierbaren Monomeren
einem Radikalstartersystem zur Polymerisationsauslösung
Polymeren zur Kohäsionsverbesserung und zur Einstellung der Viskosität
(aktiven) Füllstoffen zur Verbesserung der mechanischen Eigenschaften.

Als polymerisierbare Monomere sind neben Methylmethacrylat in Kombination mit Methacrylsäure zahlreiche weitere Systeme untersucht worden, von denen einige praktische Bedeutung gewonnen haben - vgl. hierzu J.M. Antonucci, Polymer Science and Technology <u>14</u>, 357 (1981). Auch auf der Seite der Härtersysteme für bei Raumtemperatur polymerisierende Methacrylatsysteme steht heute an sich eine breite Palette von Beschleunigern zur Verfügung - vgl. beispielsweise G.M. Brauer, Org. Coat. Plast. Chem. <u>42</u>, 321 (1980) sowie J.W. Prane, Org. Coat. Plast. Chem. <u>40</u>, 338 (1979) - gleichwohl sind auch hier Verbesserungen wünschenswert.

Bekannt ist, daß sich die Haftung von Methacrylat-Klebstoffen auf Knochenmaterial durch Boralkylhärter steigern läßt. Boralkylverbindungen z.B. Triäthyl-, Tripropyl- und Tributylbor lösen in Gegenwart von Luftsauerstoff radikalische Polymerisationen aus. Sie eignen sich demzufolge auch als Härter für 2-Komponenten-Methacrylatklebstoffe vgl. hierzu JA-OS 42 14 318 und 45 29 19S sowie DE-OS 23 21 215. Die JA-OS 42 14 318 schlägt Trialkylbor als Härtungsmittel für dentalmedizinische Bindemittel und Füllmaterialien vor. Mit Trialkylbor ergeben sich Festigkeiten, wie sie bisher mit anderen Härtungsmitteln nicht erreicht werden konnten. Offenbar ist Grund hierfür, daß Trialkylbor eine Pfropfpolymerisation am Kollagen auslöst - vgl. Shikai-tenbo, <u>32</u>, 609 (1968). Der Klebstoff, bzw. das Füllmaterial haften über eine kovalente chemische Bindung am Zahnbein.

Die Verwendung von Trialkylborverbindungen wie Trimethyl-, Triäthyl-, Tripropyl- und/oder Tributylbor ist jedoch mit außergewöhnlichen Sicherheitsproblemen und anwendungstechnischen Schwierigkeiten verbunden. So besitzt beispielsweise Triäthylbor eine Zündtemperatur von -20° C.

Zur Abwendung dieser gravierenden Nachteile wurde vorgeschlagen, die Trialkylborverbindungen mit Aminen oder mit definierten Sauerstoffmengen umzusetzen - vgl. die JA-OS 45-29 195 und die DE-OS 23 21 215. Die Selbstentzündlichkeit dieser Systeme wird dadurch jedoch nicht verhindert. Die Zündtemperatur wird lediglich in den Temperaturbereich von 0 bis 70° C verschoben. Hierdurch ist nach wie vor die Herstellung größerer

Klebstoffmengen unmöglich und ihr Anwendungsbereich eingeschränkt.

Zur Kohäsionsverbesserung, zur Einstellung anwendungstechnisch günstiger Viskositäten und zur Verringerung der Volumenkontraktion während des Härtens ist es bekannt, dem Monomeren zusätzlich Polymere beizumischen, z.B. Polymethylmethacrylat, Polychloropren, chlorsulfoniertes Polyäthylen, Nitrilkautschuke und/ oder Polyurethane - vgl. US-PSen 3 333 025, 3 725 504, 3 832 274, 3 890 407 und 3 994 764.

Zur Verbesserung der Härte und Formstabilität hat es sich weiterhin als günstig herausgestellt, Füllstoffe in feinverteilter Form zuzugeben, kristalline Systeme sind dabei aufgrund ihrer hohen Packungsdichte amorphen Füllstoffen deutlich überlegen, siehe hierzu A.K. Abell et al. Pol. Sci. and Technology 14, 347 (1981).

Sowohl die im Klebstoffsystem eingesetzten Monomeren als auch das jeweils verwendete Startersystem beeinflussen die Festigkeit der Klebeverbindung. Bei der Verklebung von hartem Gewebe in der Medizin hängt die erzielte Festigkeit zusätzlich stark von der Vorbehandlung des Knochenmaterials und den Lagerbedingungen der gefügten Teile ab. Für die Verwendung von Klebstoffsystemen im Organismus sind Festigkeitsmessungen auf entfettetem trockenem Knochengewebe wenig aussagekräftig. Relevanter erscheint die Verklebung von nicht vorbehandelten feuchten und fetten Knochen und die Festigkeitsmessung an den erhaltenen Prüfkörpern nach Lagern in (Blut)-Ringer-Lösung. Unter diesen simulierten in-vivo-Bedingungen erzielten konventionelle Methacrylatklebstoffe und Knochenzemente auf Knochenmaterial Zugfestigkeiten von ca. 60 $Ncm^{-2}$ (vergleiche hierzu G. Giebel et al. Biomed. Techn. 26, 170 (1981)). Diese Festigkeitswerte sind für eine Reihe von Anwendungsgebieten, beispielsweise für die Vereinigung von Knochenfragmenten mit dem Ziel einer sofortigen Belastbarkeit nach dem Aushärten des Zements bzw. Klebstoffes deutlich zu niedrig. Hieraus resultieren die eingangs geschilderten Beschränkungen in der Anwendbarkeit von Knochenzement.

Bekannt ist schließlich, daß bestimmte Kunststoffmassen im lebenden Organismus abgebaut werden. Handelsüblich ist beispielsweise im Organismus resorbierbares Fadenmaterial auf Basis von Polyglycolsäure oder Polymilchsäure. Auch Anwendungen dieses Materials in der Zahnmedizin und Orthopädie sind bekannt. Zur Abbaubarkeit der Polymeren liegt umfangreiches Datenmaterial vor, siehe beispielsweise R.A. Miller et al. J.Biomed, Mat. Res. 1977, 11, 711-719; D.C. Miln et al, Scot. med. I, 17, 108 (1972) sowie A.M. Reed et al. Polymer 24, 499 (1981). Durch Co-Kondensation von Glycolsäure und Milchsäure kann die Abbaugeschwindigkeit innerhalb weiter Grenzen wunschgemäß eingestellt werden, vgl. die zuvor zuerst genannte Literaturstelle.

Polyglycolsäure wird üblicherweise aus Glycolsäure über die Zwischenstufe des Glycolids mit geeigneten Katalysatoren bei Temperaturen im Bereich von 180 bis 200° C hergestellt. Die direkte Polykondensation von Glycolsäure bei 118° C und 5 mbar führt zu oligomeren Produkten niedriger Kohäsion - siehe A.M. Reed et al. aaO.

Für die Anwendung in der Medizin als Klebstoff oder Zement kommen Polyglycolsäure und/oder Polymilchsäure bisher nicht in Frage. Klebstoffe und Zemente sind durch die Applikation einer flüssigen Form gekennzeichnet, die nach der Anwendung zum festen Polymer führt. Aufgrund der hohen benötigten Reaktionstemperaturen ist die in-situ-Erzeugung von Polyglycolsäuren und Polymilchsäuren im Organismus zur Verbindung von Substraten nicht möglich.

## Aufgabe der Erfindung

Aufgabe der vorliegenden Erfindung ist die Schaffung von Klebstoffen bzw. selbsthärtenden Kunststoffmassen für die Verwendung in der Medizin, die sich ins besondere durch die Kombination der folgenden Eigenschaften auszeichnen:

Hohe Kohäsion und gleichzeitig gute Adhäsion auf feuchten und fettigen Oberflächen sowie Abbaubarkeit der gehärteten Kunststoffmassen im Organismus, insbesondere mit beeinflußbarer Geschwindigkeit des Abbaus.

Im weiteren Sinne ist damit Aufgabe der Erfindung, für das chirurgische Arbeiten im Bereich der Human-und Tiermedizin härtbare Kunststoffmassen zur Verfügung zu stellen, die als Klebstoffsysteme zur Verklebung von körpereigenem Hartgewebe beispielsweise Knochen - gewünschtenfalls auch mit körperfremden Materialien, wie Kunststoff oder Metall - geeignet sind. Diese härtbaren Kunststoffmassen sollen aber auch zur in-situ-Herstellung von individuell gestalteten Formteilen geeignet sein.

## Gegenstand der Erfindung

Gegenstand der Erfindung ist dementsprechend in einer ersten Ausführungsform ein zum Verkleben von körpereigenem Hartgewebe geeignetes chirurgisches Bindemittelsystem auf Basis eines polymerisierbaren (Meth)-acrylatklebers und eines polymerisationsauslösenden Starters, wobei dieses System dadurch gekennzeichnet ist, daß es als resorbierbare (Meth)acrylatkomponente bei Raumtemperatur flüssige bis feste (Meth)-acrylsäureester mit (Meth)-acrylatresten an aus Hydroxycarbonsäuren gebildeten Polyester-Oligomerketten enthält und wobei weiterhin als Reaktionsauslöser für die Polymerisations- bzw. Härtungsreaktion Organoborverbindungen vorgesehen sind. Gegenstand der Erfindung ist weiterhin der Einsatz solcher Bindemittelsysteme zum Verkleben von körpereigenem Hartgewebe, beispielsweise Knochen miteinander, gewünschtenfalls aber auch mit fremden Materialien wie Kunststoff oder Metall.

Ein weiterer Gegenstand der Erfindung betrifft die Verwendung von Bindemittelsystemen der geschilderten Art zur in-situ-Herstellung von individuell ausgebildeten Formteilen, insbesondere im Zusammenhang mit der Verklebung von körpereigenem Hartgewebe, gegebenenfalls zusammen mit Kunststoff und/oder Metall.

**Polymerisierbare (Meth)-acrylat-Komponente**

Als ein wesentlicher Bestandteil der erfindungsgemäßen Bindemittelsysteme werden reaktive Monomere auf Acrylat- bzw. Methacrylatbasis - im Rahmen der Erfindungsbeschreibung als (Meth)-acrylatverbindungen bezeichnet - eingesetzt, wobei insbesondere den entsprechenden Methacrylatverbindungen besondere Bedeutung zukommt. Diese bisher nicht vorbeschriebenen reaktiven (Meth)-acrylatverbindungen sind Gegenstand der älteren deutschen Patentanmeldung D 6529 (P 32 04 504.2). Sie zeichnen sich bei der Anwendung in Klebstoffen durch eine Mehrzahl wünschenswerter Eigenschaften aus. Sie besitzen beispielsweise aufgrund ihres niedrigen Dampfdrucks eine geringe Flüchtigkeit. Trotz vergleichsweise hohem Molekulargewicht besteht die Möglichkeit, sie bei Raumtemperatur als Flüssigkeiten oder wenigstens viskos streichbare Pasten auszubilden. Sie besitzen gute Mischbarkeit mit den üblicherweise eingesetzten weiteren Komponenten der hier betroffenen Bindemittelsysteme. Es lassen sich mit ihnen hoch-feste elastische Klebeverbindungen erzielen. Insbesondere führen diese erfindungsgemäß ausgewählten reaktiven Monomeren aber zu ausgehärteten Kunststoffen, die vom lebenden Organismus resorbiert werden können. Ihr Einsatz im Rahmen chirgurgischer Arbeiten führt damit zur reversiblen Ausbildung von Verklebungen, Stütz— bzw. Halterungselementen und dergleichen, die beispielsweise bei Knochenfrakturen eine zeitlich begrenzte Wirkung entfalten, letztlich aber wieder resorptiv abgebaut werden.

Die erfindungsgemäß eingesetzten (Meth)-acrylat-Derivate enthalten einen oder vorzugsweise mehrere (Meth)-acrylatreste an einer oligomeren Polyesterkette, die aus Hydroxycarbonsäuren gebildet worden ist. Bevorzugt sind dabei solche (Meth)-acrylatverbindungen, die zwei (Meth)-acrylatreste an dem Polyester-Oligomersegment aufweisen. Diese Verbindungen können als solche eingesetzt werden. Es fallen aber auch Mischungen verschiedener Verbindungstypen in den Rahmen der Erfindung, insbesondere solche aus entsprechenden Komponenten mit nur einer (Meth)-acrylat-Gruppe und solchen mit zwei (Meth)-acrylat-Gruppen am Polyester-Oligomer-Segment.

In den für die praktische Anwendung wichtigeren Typen mit zwei (Meth)-acrylatresten im Molekül sitzen bevorzugt diese funktionsfähigen Gruppen derart endständig am Oligomer-Segment, daß die beiden Endeinheiten des Oligomeren-Segments jeweils mit einer (Meth)-acrylat-Gruppe substituiert sind ($\alpha,\omega$ -Stellung).

Die Oligomersegmente weisen das Strukturmerkmal

$$-\left[ O - R - \overset{\overset{\displaystyle O}{\|}}{C} \right]_n-$$

auf. Sie sind durch Oligomerisierung geeigneter Hydroxycarbonsäuren bzw. Hydroxycarbonsäuregemische der allgemeinen Formel

$$HO - R - \overset{\overset{\displaystyle O}{\|}}{C} - OH$$

zugänglich.

In einer bevorzugten Ausführungsform der Erfindung sind die Reste -R- und n derart ausgewählt, bzw. aufeinander abgestimmt, daß das mittlere Molekulargewicht der Polyester-Oligomereinheit im Bereich von etwa 200 bis 600 liegt. Besonders bevorzugte Werte für das mittlere Molekulargewicht liegen im Bereich von etwa 300 bis 500.

Das Polyester-Oligomersegment wird in einer bevorzugten Ausführungsform der Erfindung aus Monohydroxymonocarbonsäuren gebildet, die eine C-Zahl von etwa 20 im Molekül und insbesondere von etwa 10 nicht überschreiten. Polyester-Oligomere aus entsprechenden Säuren mit 2 bis 10, insbesondere 2 bis 7 Kohlenstoff-Atomen im Molekül können besonders wichtig sein. Es können dabei zur Ausbildung des Polyester-Oligomeren bestimmte ausgewählte einzelne Hydroxycarbonsäuren oder aber auch Gemische verschiedener Hydroxycarbonsäuren zur Verwendung kommen. Besonders wichtige Hydroxycarbonsäuren zur Ausbildung dieses Mittelstücks der erfindungsgemäß eingesetzten (Meth)-acrylatverbindungen sind Glycolsäure, die isomeren Milchsäuren, die gegebenenfalls isomeren $\alpha$- oder $\beta$-Hydroxypropionsäuren, die gegebenenfalls

isomeren α-, β- oder γ -Hydroxybuttersäuren, o-Hydroxybenzoesäure (Salicylsäure), m-Hydroxybenzoesäure und/oder p-Hydroxybenzoesäure (Anissäure). Es können dabei sowohl bestimmte Isomere der genannten Säuren als auch beliebige Gemische zum Einsatz kommen.

Die Polyester-Oligomeren können dabei zweckmäßiger-weise unter Mitverwendung von monofunktionellen und/oder vorzugsweise unter Mitverwendung von difunktionellen Reaktanten hergestellt worden sein, die eine mehrfache Funktion erfüllen. Einerseits wird durch diese mitverwendeten Reaktanten die Regelung des mittleren Molekulargewichts im Polyester-Oligomeren und damit die Einstellung der angestrebten Viskositätsbereiche ermöglicht. Zum anderen ist durch Auswahl der funktionellen Gruppen der mitverwendeten Co-Reaktanten die Möglichkeit gegeben, an den Polyester-Oligomeren einheitlich endständige Hydroxylgruppen oder endständige Carboxylgruppen auszubilden, was an sich bei einem Polymeren bzw. Oligomeren einer Hydroxycarbonsäure zunächst ja nicht der Fall ist. Schließlich gelingt es durch Mitverwendung ausgewählter monofunktioneller Co-Reaktanten eine reaktive Endgruppe der Polyester-Oligomeren zu beseitigen, so daß an diesem Typus von Verbindungen für die nachfolgende Anbindung der (Meth)-acrylatreste nur eine reaktive Funktion zur Verfügung steht. Durch geeignete Abmischung von monofunktionellen und difunktionellen Co-Reaktanten ist es schließlich möglich, vorbestimmte Mischungsverhältnisse von Mono-(meth)-acrylatverbindungen und Bis-(meth)-acrylatverbindungen einzustellen.

Als monofunktionelle Co-Reaktanten kommen insbesondere Monoalkohole, Monocarbonsäuren und/oder Monoamineprimäre, sekundäre und/oder tertiäre Monoamine - in Betracht. Difunktionelle Co-Reaktanten bei der Herstellung der Polyester-Oligomeren sind difunktionelle Alkohole oder Dicarbonsäuren bzw. funktionelle reaktive Dicarbonsäurederivate, beispielsweise entsprechende Anhydride, Ester, Halogenide und dergleichen.

Werden Oligomere der geschilderten Art durch Cokondensation von Hydroxycarbonsäuren und Diolen hergestellt, so entstehen letztlich Polyester-Oligomere mit endständigen Hydroxylgruppen. Die Menge der mitverwendeten Diole bestimmt - in Abstimmung mit den Reaktionsbedingungen - das mittlere Molekulargewicht des anfallenden Polyester-Oligomeren. Polyester-Oligomere mit Hydroxylendgruppen können leicht durch Umsetzung mit Säureanhydriden in analoge Derivate mit Carboxylendgruppen überführt werden. Werden andererseits die Oligomeren durch Cokondensation von Hydroxycarbonsäuren mit Dicarbonsäuren beziehungsweise reaktiven Dicarbonsäurederivaten hergestellt, so entstehen direkt Polyester-Oligomere mit endständigen Carboxylgruppen beziehungsweise Carboxylgruppenderivaten. Auch hier wirkt der mitverwendete Co-Reaktant gleichzeitig vereinheitlichend bezüglich der endständigen reaktiven Gruppen und molekulargewichtsregelnd. Im einzelnen gilt hier das bekannte Wissen zur Herstellung von Polyestern beziehungsweise Copolyestern. Die Mitverwendung von Monoalkoholen und/oder Monoaminen führt zur bevorzugten Blockierung der endständigen Carbonsäuregruppierung im Polyester-Oligomeren, die Mitverwendung von Monocarbonsäuren blockiert dementsprechend die endständige Hydroxylgruppenseite des Oligomeren.

In allen hier geschilderten Fällen entstehen modifizierte Polyester-Oligomere, die in an sich bekannter Weise einfach zu den erfindungsgemäß zu verwendenden (Meth)-acrylatverbindungen umgesetzt werden können. Liegen endständige Hydroxylgruppen am Polyester-Oligomeren vor, so wird die (Meth)-acrylsäuregruppe durch Veresterung beziehungsweise Umesterungbzw. durch vergleichbare Reaktion - mit Acrylsäure oder Acrylsäureestern und/oder insbesondere entsprechenden Methacrylsäureverbindungen eingeführt. Liegen im Polyester-Oligomeren jedoch endständige Carboxylgruppen vor, so lassen sich ebenfalls in an sich bekannter Weise leicht die gewünschten (Meth)acrylatgruppen anbinden. Geeignet ist hier beispielsweise die Umsetzung des oligomeren Zwischenprodukts mit Glycidylestern der Acrylsäure beziehungsweise der Methacrylsäure. Unter Aufspaltung der Glycidylgruppierung wird die (Meth)-acrylatgruppe über den Glyceridrest an die intermediär gebildete Mono- beziehungsweise Dicarbonsäure angebunden.

In der Auswahl der in der Regel nur in untergeordneten Mengen mitverwendeten monofunktionellen oder difunktionellen Co-Reaktanten sind praktisch keine Einschränkungen gegeben. So können diese Reaktanten aliphatischer, cycloaliphatischer oder aromatischer Natur sein. Sie besitzen im allgemeinen eine Kohlenstoffzahl nicht über $C_{25}$, vorzugsweise nicht über $C_{15}$. Geeignete Diole enthalten beispielsweise 2 bis 20 C-Atome, vorzugsweise 2 bis 10 und insbesondere 2 bis 6 C-Atome im Molekül. Die gleichen Angaben gelten für die entsprechenden Dicarbonsäuren. Beispiele für geeignete Diole sind: Ethylenglycol, Di-, Tri-, Tetraethylenglycol, Propylenglycol, Dipropylenglycol, 1,3-Butylenglycol, 1,4-Butylenglycol, 2,2-Dimethyl-1,3-propandiol, 2,2,4-Trimethyl-1,6-hexandiol, 1,3-Cyclohexandimethanol, 1,5-Pentandiol, 1,6-Hexandiol, 1,10-Decandiol und 2,2-Bis-(3-hydroxycyclohexyl)propan. Zur Überführung der Oligoester-Diole in Oligoester-Dicarbonsäuren eignen sich insbesondere folgende Anhydride: Bernsteinsäureanhydrid, Glutarsäureanhydrid, Phthalsäureanhydrid, Itaconsäureanhydrid, Citraconsäureanhydrid ebenso wie Alkenylbernsteinsäureanhydride.

Geeignete Dicarbonsäuren sind beispielsweise: Oxalsäure, Malonsäure, Bernsteinsäure, Glutarsäure, Adipinsäure, Pimelinsäure, Korksäure, Azelainsäure, Sebazinsäure, Isosebazinsäure, Nonandicarbonsäure, Decandicarbonsäure, Undecandicarbonsäure, Dodecandicarbonsäure, Phthalsäure, Hexahydrophthalsäure, Isophthalsäure, Terephthalsäure und Biphenyldicarbonsäure.

Als monovalente Co-Reaktanten kommen aliphatische, cycloaliphatische oder aromatische Monoalkohole, entsprechende Monocarbonsäuren und gegebenenfalls entsprechende primäre, sekundäre oder tertiäre Amine in Betracht. Ihre C-Zahl kann innerhalb der angegebenen Grenzen liegen.

Selbstverständlich können in allen Fällen - das heißt sowohl beiden Hydroxycarbonsäuren als auch bei den Co-Reaktanten - nicht nur die jeweils freien reaktiven Komponenten der genannten Art, sondern auch solche reaktive Derivate eingesetzt werden, die in an sich bekannter Weise unter den Bedingungen der Veresterung

beziehungsweise Umesterung die gewünschten Polyester-Oligomeren des vorbestimmten Molekulargewichts bilden. Geeignet sind also beispielweise die Ester der Hydroxycarbonsäuren, sowie Lactone beziehungsweise Lactame von Hydroxycarbonsäuren, die mit Diolen beziehungsweise Diolestern umgesetzt, insbesondere umgeestert werden können. Sowohl die Herstellung der Polyester-Oligomeren als deren Umsetzung zu den (Meth)-acrylatverbindungen erfolgt in an sich bekannter Weise zum Beispiel durch Reaktion in Gegenwart oder in Abwesenheit von Lösungsmitteln, gewünschtenfalls in Gegenwart von Katalysatoren, insbesondere Veresterungskatalysatoren.

Werden Mischungen von difunktionellen und monofunktionellen (Meth)-acrylatverbindungen eingesetzt, so liegt in der bevorzugten Ausführungsform der Gehalt monofunktioneller Komponenten nicht oberhalb von 95 Mol-Prozent, bezogen auf die Mischung der monofunktionellen und der difunktionellen Komponenten. Bevorzugt sind Gemische der genannten Art, in denen der Gehalt an monofunktioneller Komponente nicht über 50 Mol-Prozent und insbesondere nicht oberhalb von 10 Mol-Prozent liegt.

In einer bevorzugten Ausführungsform der Erfindung werden (Meth)-acrylatverbindungen beziehungsweise entsprechende Gemische eingesetzt, die bei Raumtemperatur fließfähig oder wenigstens noch pastös verstreichbar sind, so daß sie als Hauptkomponente oder sogar als alleinige polymerisierbare Klebstoffkomponente in den chirurgischen Bindemitteln eingesetzt werden können. Es kann dabei bevorzugt sein, daß die flüssigen (Meth)-acrylatverbindungen bei Raumtemperatur eine Viskosität im Bereich von etwa 500 bis 70.000 mPas vorzugsweise im Bereich von etwa 3000 bis 50.000 mPas besitzen. Feste (Meth)-acrylatverbindungen der erfindungsgemäß verwendeten Art lösen sich jedoch gut in flüssigen polymerisierbaren Komponenten, beispielsweise in Methylmethacrylat, so daß also auch feste Verbindungen der Erfindung in Abmischung mit flüssigen konventionellen Monomerbestandteilen in einfacher Weise zu den verarbeitungstechnisch gewünschten, flüssigen Klebstoffkomponenten aufgearbeitet werden können.

**Organoborverbindungen als Initiator-Komponente**

Als Initiator-Komponente für die Polymerisation und damit Aushärtung der Bindemittelsysteme kommen Organoborverbindungen in Betracht, die Gegenstand einer Reihe älterer Schutzrechtsanmeldungen der Anmelderin sind. Gemeinsam ist diesen Initiatoren auf Basis reaktiver Borkomponenten: Sie besitzen eine vergleichbare Reaktivität wie die bekannten niederen Boralkyle, zeigen jedoch gleichzeitig eine deutlich verringerte Neigung zur Selbstentzündlichkeit. Damit wird eine leichte und ungefährliche Handhabung ermöglicht. Die erfindungsgemäß einzusetzenden Organoborverbindungen zeigen bei der Anwendung an der Luft nur einen langsamen Aktivitätsverlust. Sie können als getrennte Härterkomponente in Zweikomponentenklebstoff-Systemen zum Einsatz kommen, möglich ist insbesondere aber auch ihre Anwendung als Primer in Systemen der sogenannten No-mix-Klebstoffe. Die erfindungsgemäß eingesetzten borhaltigen Härter führen zu hohen Festigkeiten der Klebstoffbindungen beziehungsweise der in situ gebildeten geformten Kunststoffteile. Gleichzeitig besteht die Möglichkeit die Härtungsgeschwindigkeit zu beeinflussen und damit den in der Praxis gewünschten Verhältnissen anzupassen. Die Struktur der Initiator-Komponente gewährleistet schließlich gute Adhäsion des Klebstoffs beziehungsweise Bindemittels an körpereigenem Material.

Erfindungsgemäß verwendbare Härter auf Basis von Organoborverbindungen sind beispielsweise geschildert in den offengelegten europäischen Patentanmeldungen No. 81109074.5 (D 6144 EP), No. 81109073.7 (D 6246 EP).

Besondere Bedeutung haben allerdings die bororganischen Verbindungen beziehungsweise Systeme, die in den älteren deutschen Patentanmeldungen D 6391 (P 31 43 945.4), D 6460 (P 32 01 780.4), D 6461 (P 32 01 731.6), D 6517 (P 32 07 264.3) und D 6548 (P 32 07 263.5) beschrieben sind.

Geeignete Initiatoren für die neuen Systeme können damit aus der folgenden Gruppe von Borverbindungen ausgewählt sein:

a) Borverbindungen mit sterisch hindernden Alkylgruppen der allgemeinen Formeln

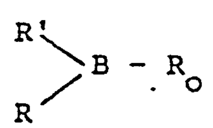

und

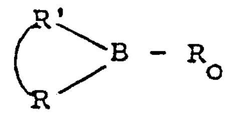

wobei R' beziehungsweise R für einen aliphatischen Mono- oder Dicyclus mit 3 bis 25 Kohlenstoffatomen stehen und $R_o$ entweder H oder einen gegebenenfalls cyclischen Kohlenwasserstoffrest mit 1 bis 15 Kohlenstoffatomen darstellt,

b) Borverbindungen, die Umsetzungsprodukte von Dihydroxyaromaten mit $BH_3$ beziehungsweise deren Alkylierungsprodukte sind, mit der allgemeinen Formel

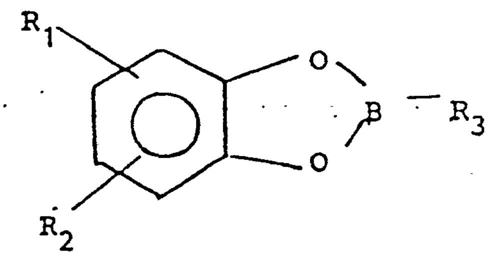

wobei $R_1$, $R_2$ und $R_3$ entweder H oder einen Alkylrest mit 1 bis 4 Kohlenstoffatomen darstellen und $R_1$ und $R_2$ auch einen aromatischen und/der aliphatischen Cyclus bedeuten können,

c) Borverbindungen der allgemeinen Formel

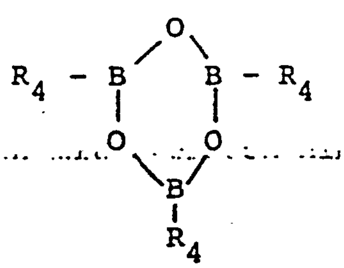

wobei $R_4$ einen Alkylrest mit 1 bis 6 Kohlenstoffatomen darstellt.

Als Borverbindungen kommen demnach zahlreiche bekannte beziehungsweise auf bekannte Weise herstellbare Boralkyle in Frage. Typische Vertreter solcher Borverbindungen sind beispielsweise 9-Borabicyclo-[3-3.1]-nonan, Diisopinocampheylboran, Dicyclohexylboran, Thexylboran-(2,3-dimethyl-2-butylboran), 3,5-Dimethylborinan, Diisoamylboran. Unter diesen Verbindungen ist das zuerst genannte 9-Borabicyclo-[3.3.1]-nonan aus praktischen Gründen bevorzugt.

Eine Zusammenstellung der Herstellungsmöglichkeiten geeigneter Borverbindungen findet sich in der Monographie Herbert C. Brown, 1975 "Organic Synthesis via Boranes", Verlag John Wiley & Sons. Als Initiatoren können weiterhin Hydroborierungsprodukte von Dialkylboranen und Olefinen eingesetzt werden. Als Olefine sind denkbar Buten, Isobuten, Hexen, Cyclohexen, Vinylchlorid, Allylchlorid, Allylamin oder auch Methacrylsäuremethylester, Vinylacetat oder Crotonsäuremethylester. Unter den geeigneten Verbindungen sind beispielsweise erwähnenswert: Diisopinocampheylbutylbor, Thexylcyclohexylcyclopentylbor, Thexyllimonylbor, Trinorbornylbor, B-Butyl-9-borabicyclo [3.3.1] nonan, B-Isobutyl-9-borabicyclo [3.3.1] nonan, B-Isobutyl-9-borabicyclo [3.3.1] nonan, B- [2-(4-Cyclohexenyl)ethyl] 9-borabicyclo [3.3.1] nonan, B-Cyclopropyl-9-borabicyclo [3.3.1] nonan, B-p-Tolyl-9-borabicyclo [3.3.1] nonan, B-tert.-Butyl-3,5-dimethylborinan. Des weiteren sind Umsetzungsprodukte von 1,2-Dihydroxybenzolen wie Brenzcatechin mit Borwasserstoff (Catecholboran) und Trin-butylboroxin geeignet.

Weitere besonders bevorzugte Initiatoren beziehungsweise Härter sind nachfolgend unter d) beschrieben:

d) Diese Startersysteme der Erfindung bestehen aus einer homogenen Mischung wenigstens einer durch Luftzutritt aktivierbaren Organoborverbindung mit wenigstens einem bei Raumtemperatur flüssigen bis festen, den Organoborverbindungen gegenüber inerten organischen Oligomeren beziehungsweise Polymeren.

Unter dem Begriff der "homogenen Mischung" werden dabei bevorzugt bei Lagerungs- und Anwendungstemperatur einphasige Stoffgemische verstanden, wie sie charakteristisch für echte Lösungen sind.

Als Organoborverbindungen sind in erster Linie Boralkyl- und/oder Borarylverbindungen beziehungsweise die entsprechenden Organoborhydridverbindungen geeignet. Boralkylverbindungen beziehungsweise Boralkylhydride sind eine besonders geeignete Stoffklasse. In Betracht kommen also in der bevorzugten Ausführungsform Verbindungen der Typen:

$R_1R_2R_3B$,

$R_1R_2BH$ und/oder $R_1BH_2$,

wobei in diesen allgemeinen Formeln die Reste $R_1$, $R_2$ und $R_3$ Kohlenwasserstoffreste insbesondere Alkylreste sind, die allerdings auch Heteroatome — insbesondere 0, N und/oder S - enthalten können. Liegen wenigstens zwei solche anorganische Reste am jeweiligen Boratom vor, so können sie ihrerseits zu einem Ringsystem geschlossen sein. Die das Bor substituierenden Kohlenwasserstoffreste weisen in einer bevorzugten Ausführungsform jeweils nicht mehr als etwa 30 C-Atome, vorzugsweise nicht mehr als etwa 25 C-Atome auf. Es kann bevorzugt sein, daß jeder dieser organischen Reste am Bor nicht mehr als etwa 12 bis 15 C-Atome besitzt.

Als borhaltige Initiatorkomponente oder auch zur Herstellung geeigneter borhaltiger Initiatorkomponenten können insbesondere Organobor-mono-hydridverbindungen, vor allem Dialkylbormonohydride Verwendung finden.

Ein typischer Vertreter solcher Borverbindungen ist beispielsweise das zuvor unter a) genannte 9-Borabicyclo [3.3.1] nonan (9-BBN), das aus praktischen Gründen bevorzugt sein kann.

Als Bor-Initiatoren können in dieser Ausführungsform aber auch einfache Trialkylborane sowie Hydroborierungsprodukte von Mono- oder insbesondere Dialkylboranen und Olefinen eingesetzt werden, wie sie zuvor beschrieben worden sind.

Als Lösungsmittel mit sehr niedrigem Dampfdruck eignen sich Oligomere beziehungsweise Polymere, die gegenüber den Organoborverbindungen inert sind, im übrigen aber keinen Einschränkungen bezüglich ihrer Struktur unterliegen, vorausgesetzt, es besteht die homogene Mischbarkeit zwischen diesen als Lösungsmittel wirkenden Polymersubstanzen und den Organoborverbindungen. Geeignet sind dementsprechend alle Polymerisate, Polykondensate und/oder Polyadditionsprodukte, die die angegebenen Bedingungen erfüllen. Die durchschnittlichen Molekulargewichte dieser oligomeren beziehungsweise polymeren Lösungsmittel können im Bereich von 200 bis 50.000.000 g/Mol liegen. Je nach Struktur und Molekulargewicht können diese Lösungsmittel bei Raumtemperatur niedrig-viskos fließfähig bis fest sein. Es kann wünschenswert sein, daß die Organoborverbindungen enthaltenden Stoffmischungen bei Raumtemperatur viskos fließfähig beziehungsweise streichfähig sind. Für die Wirksamkeit der erfindungsgemäß eingesetzten Startersysteme als Initiatoren ist das allerdings keine Voraussetzung. Im Gegenteil kann die Lagerstabilität von entsprechenden bei Raumtempeatur festen Stoffmischungen besonders gut sein.

Geeignete polymere Lösungsmittel im hier betroffenen Sinne sind beispielsweise entsprechende Polyether, Polyester, Polyamide, Polyurethane, Polysiloxane und dergleichen. Für die Herstellung fließfähiger Systeme können bei Raumtemperatur flüssige Oligomere mit Viskositäten etwa im Bereich von 1.000 bis 70.000 mPas (Raumtemperatur) besonders interessant sein. Es hat sich gezeigt, daß insbesondere Polyester und Polyamide im Rahmen der Erfindung vielseitig anwendbar sind. Sie können - ebenso wie die anderen polymeren Lösungsmittel - in an sich bekannter Weise hergestellt werden, im hier betroffenen Fall beispielsweise durch Polykondensation von Dicarbonsäuren mit Diolen beziehungsweise Diaminen gegebenenfalls unter Mitverwendung von monofunktionellen Reaktanten zur Einschränkung beziehungsweise Regulierung des mittleren Molekulargewichts. Bevorzugt sind hier im allgemeinen gesättigte Dicarbonsäuren und gesättigte Glycole mit jeweils bis zu 15 C-Atomen insbesondere mit bis zu 10 C-Atomen im Molekül. Analog gelten diese Angaben auch für die Amine beziehungsweise Diamine zur Herstellung von Polyamiden. Geeignete Polyether sind beispielsweise Polyethylenoxide oder Polypropylenoxide mit Molekulargewichten im angegebenen Bereich. Im einzelnen gilt hier das umfangreiche Fachwissen der Polymerchemie.

Zur Herstellung der Startersysteme werden die Organoborverbindungen unter völligem Sauerstoffaus-schluß in den inerten organischen Oligomeren beziehungsweise Polymeren gelöst. Gegebenenfalls kann dabei unter mäßigem Erwärmen gearbeitet werden. So ist es beispielsweise möglich, zur Beschleunigung der Auflösung auf Temperaturen bis zu 100° C vorzugsweise etwa 70 °C zu erwärmen. Bei der Verwendung von bei Raumtemperatur festen Oligomeren beziehungsweise Polymeren ist die Mitverwendung inerter flüssiger Lösungsmittel zweckmäßig. Geeignet sind hier die bekannten Lösungsmittel für Organoborverbindungen insbesondere Tetrahydrofuran oder Polyether, wie Diethylenglycoldimethylether aber auch Ester, Halogenkohlenwasserstoffe und dergleichen. Unter Mitverwendung dieser flüssigen Hilfsmittel werden innige Mischungen hergestellt, die Hilfsflüssigkeiten können dann abgezogen und dann die Boralkyl/Oligomer-Gemische isoliert werden. Ihre Lagerung erfolgt zweckmäßigerweise im verschlossenen Gefäß, bevorzugt unter Inertgas, beispielsweise unter Stickstoff.

Der Gehalt an Organoborverbindungen in solchen Startersystemen liegt üblicherweise nicht über etwa 70 Gewichtsprozent und vorzugsweise nicht über etwa 50 Gewichtsprozent- jeweils bezogen auf das

Gesamtgewicht der Mischung. Es ist weiterhin bevorzugt, daß der Gehalt an Organoborverbindung wenigstens etwa 1 Gewichtsprozent des Gesamtsystems ausmacht, so daß Mengen von etwa 1 bis 50 Gewichtsprozent insbesondere 3 bis 50 Gewichtsprozent besonders geeignet sein können.

Eine weitere für die Praxis wichtige Möglichkeit benutzt als Initiator-Komponente gegen Luftzutritt stabilisierte polymere Organoborverbindungen, die im folgenden unter e) geschildert werden:

e) Das Kennzeichen dieser Klasse bororganischer Härter liegt darin, daß hier mit polymeren Organoborverbindungen gearbeitet wird, die als Substituenten an einer gegen Luftzutritt stabilen Polymer: matrix Borwasserstoff- und/oder Organoborreste aufweisen.

Diese borhaltigen Reste sind vorzugsweise über B-C-Bindungen an die Polymermatrix gebunden. Sofern die borhaltigen Reste nicht den Boranrest $-BH_2$ selber darstellen, sind die borhaltigen Substituenten der Polymermatrix in einer weiterhin bevorzugten Ausführungsform ihrerseits am Bor mit wenigstens einer weiteren B-C-Bindung an einen oder mehrere organische Reste gebunden. Bevorzugte Reste sind hier Kohlenwasserstoffreste, die allerdings auch Heteroatome insbesondere O, N und/oder S enthalten können. Geeignete Substituenten am Bor sind insbesondere Alkyl-, Cycloalkyl- und/oder Arylreste, die in einer oder in den beiden nicht durch die Polymerimatrix besetzten Valenzen des Bors vorliegen können. Liegen solche von Wasserstoff abweichenden organischen Reste in beiden Borvalenzen vor, so können sie ihrerseits zu einem Ringsystem geschlossen sein.

Gegenüber herkömmlichen Boralkylhärtern besitzen die oligomeren bzw. polymeren Borverbindungen wesentliche Vorteile. Sie sind nicht selbstentzündlich und stellen geringe Anforderungen bei der Lagerung. Die Aktivität dieser Härter bleibt selbst bei längerer Lagerung an der Luft erhalten. Die Verträglichkeit der polymerisierbaren Komponente mit dem Härter kann durch geeignete Ausgestaltung der Polymermatrix für jeden Fall leicht sichergestellt werden. Im allgemeinen ist die zur Härtung der Monomerkomponente benötigte Menge der oligomeren bzw. polymeren Organoborverbindung äußerst niedrig.

Diese polymeren Borverbindungen können in einfacher Weise erhalten werden, indem Oligomere bzw. Polymere, die additionsbereite Kohlensotff-Doppelbindungen enthalten, der Hydroborierung unterworfen und hierdurch die borhaltigen Reste an wenigstens einem Teil der additionsbereiten Doppelbindungen eingeführt werden. Zur Hydroborierung geeignet sind sowohl das Diboran als auch mono- oder disubstituierte Borane, d.h. Verbindungen der allgemeinen Formel $R_4R_5BH$, wobei in dieser Formel $R_4$ ein organischer Rest, bevorzugt ein Kohlenwasserstoffrest, und $R_5$ Wasserstoff oder ebenfalls ein organischer Rest ist, der mit $R_4$ gleich oder von ihm verschieden sein kann oder auch mit $R_4$ und dem Bor gemeinsam ein Ringsystem bildet.

Die der Hydroborierung zugängliche ethylenische Doppelbindungen aufweisende Polymermatrix kann je nach Struktur und Molekulargewicht niedrigviskos fließfähig bis fest sein. Ihr mittleres Molekulargewicht kann bis zu Werten von einigen Millionen reichen und liegt üblicherweise im Bereich von etwa 150 bis 3 Millionen. Niedrigere Werte innerhalb dieses Bereiches sind häufig bevorzugt, beispielsweise solche im Bereich von etwa 300 bis 500.000 und insbesondere solche im Bereich von etwa 500 bis 10.000. Auch hier kann es wünschenswert sein, daß die Polymermatrix und auch die daraus gewonnenen polymeren Organoborverbindungen bei Raumtemperatur viskos fließfähig bzw. streichfähig sind. Hier können beispielsweise Molekulargewichte der Polymermatrix im Bereich von etwa 300 bis 3000 besonders geeignet sein. Für die Wirksamkeit der erfindungsgemäß eingesetzten polymeren Organobor-Verbindungen als Initiatoren ist das keine Voraussetzung. Im Gegenteil kann die Lagerstabilität Von bei Raumtemperatur festen entsprechenden polymeren Organoborverbindungen besonders gut sein.

Die polymere Matrix kann vor der Hydroborierung in beliebig starkem Ausmaß ethylenisch ungesättigt sein. Bevorzugt sind entsprechende Materialien, die vor der Hydroborierung eine Jodzahl im Bereich von etwa 1 bis 500 aufweisen. Innerhalb dieses Bereiches sind besonders bevorzugte Werte der Jodzahl von etwa 5 bis 100 und insbesondere von etwa 8 bis 50.

Die der Hydroborierung zugänglichen ethylenischen Doppelbindungen können im Ausgangspolymeren in der Hauptkette und/oder in Seitenketten-Substituenten vorliegen.

Die Polymermatrix kann dementsprechend geradkettige oder verzweigte Struktur aufweisen, aber auch polymere Materialien mit vernetzter Struktur sind nicht ausgeschlossen.

Als Polymermatrix eignen sich grundsätzlich alle Polymertypen, soweit sie der Hydroborierung zugängliche Doppelbindungen aufweisen und keine reaktiven Gruppen besitzen, die bei der Einführung der borhaltigen Gruppen in das Polymermaterial zu unerwünschten Nebenreaktionen führen.

Das Polymermaterial kann durch Polymerisation bzw. Co-Polymerisation olefinisch ungesättigter Komponenten, durch Polykondensation oder durch Polyaddition hergestellt worden sein. Durch an sich bekannte geeignete Auswahl der die Polymeren aufbauenden Monomertypen wird der erwünschte Gehalt an reaktiven Doppelbindungen für die anschließende Hydroborierung im Polymermaterial sichergestellt. Besonders geeignet können als Polymermatrix ungesättigte Oligomere bzw. Polymere sein, die durch Polykondensation erhalten worden sind. In Betracht kommen hier alle bekannten Polykondensattypen wie Polyester, Polyamide, Polyimide, Polycarbonate, Polyurethane und dergleichen. Geeignet sind aber auch Oligomere bzw. Polymertypen, die durch Polyaddition erhalten werden. Einzelheiten sind der älteren deutschen Patentanmeldung D 6461 (P 32 01 731.6) zu entnehmen.

Das Ausmaß der Hydroborierung an der Polymermatrix kann im Rahmen der insgesamt vorliegenden Doppelbindungen frei gewählt werden. Es hat sich allerdings als vorteilhaft erwiesen, wenn wenigstens ein substantieller Anteil dieser Doppelbindungen durch Einführung der borhaltigen Substituenten umgesetzt wird. So sind in bevorzugten Ausführungsformen der Erfindung wenigstens 30 % und vorzugsweise wenigstens 50 %

der in der Polymermatrix ursprünglich vorhandenen ethylenischen Doppelbindungen hydroboriert. Besonders geeignet sind solche polymeren Organoborverbindungen, in denen wenigstens 80 % vorzugsweise wenigstens 90 % oder sogar wenigstens 95 % der ethylenischen Doppelbindungen der Umsetzung mit den borhaltigen Komponenten zugeführt worden sind. Ein praktisch vollständig hydroboriertes Material ist in vielen Fällen der bevorzugte Initiator im Sinne der erfindungsgemäßen Lehre.

Zur Hydroborierung kommen neben Diboran ($BH_3$) Organoboranverbindungen in Betracht, die einen oder zwei organische Reste insbesondere Kohlenwasserstoffreste aufweisen. Bevorzugte organische Reste sind hier Alkyl-, Cycloalkyl- und/oder Arylreste, wobei zwei gegebenenfalls vorliegende Reste auch miteinander - unter Einschluß des Boratoms - zu einem Ring geschlossen sein können. Die substituierenden Kohlenwasserstoffreste weisen insbesondere jeweils nicht mehr als 25 C-Atome auf. Bevorzugt enthält jeder dieser Reste nicht mehr als etwa 12 bis 15 C-Atome.

Eine besonders geeignete Klasse von Organoborverbindungen für die Herstellung der polymeren Initiatorkomponente sind Organobor-monohydridverbindungen, insbesondere Dialkylmonohydride. Ein typischer Vertreter solcher Borverbindungen ist auch hier das 9:Borabicyclo [3.3.1] nonan, das aus praktischen Gründen bevorzugt ist.

Eine weitere interessante Klasse von Organobor-Initiatoren ist im folgenden unter f) geschildert:

f) In dieser Ausführungsform sind die einzusetzenden Organoborverbindungen dadurch gekennzeichnet, daß sie Borwasserstoffreste oder Organoborreste an Fettsäure- und/oder Fettalkoholestern enthalten.

Diese Boralkylverbindungen sind Hydroborierungsaddukte von Diboran und/oder Organoborverbindungen mit wenigstens einer B-H-Bindung an Fettsäure— und/oder Fettalkoholester, die wenigstens in einem Anteil der Fettsäure- und/oder Fettalkoholreste additionsbereite Kohlenstoff-Doppelbindungen enthalten.

Zur Beschaffenheit und Herstellung dieser Klasse von Boralkylverbindungen gelten im einzelnen die folgenden Angaben:

Als Matrix dienende Ester beziehungsweise Estergemische enthalten über B-C-Bindung als Substituenten Borwasserstoff- und/oder Organoborreste. Sofern diese borhaltigen Reste nicht den Boran-rest-$BH_2$ selber darstellen, sind diese borhaltigen Substituenten in einer bevorzugten Ausführungsform ihrerseits am Bor mit wenigstens einer weiteren B-C-Bindung an einen oder mehrere organische Reste gebunden. Bevorzugte Reste sind hier Kohlenwasserstoffreste, die allerdings auch Heteroatome, insbesondere O, N und/oder S enthalten können. Geeignete Substituenten am Bor sind insbesondere Alkyl-, Cycloalkyl- und/oder Arylreste, die in einer oder in den beiden nicht durch die Estermatrix besetzten Valenzen des Bors vorliegen können. Liegen solche von Wasserstoff abweichenden organischen Reste in beiden Borvalenzen vor, so können sie ihrerseits zu einem Ringsystem geschlossen sein.

Diese Borverbindungen können in einfacher Weise dadurch erhalten werden, daß man das olefinische Doppelbindungen enthaltende Ester-Matrix-Ausgangsmaterial der Hydroborierung mit Diboran oder vorzugsweise mit mono- und insbesondere disubstituierten Boranen der allgemeinen Formel $R_6R_7$ BH unterwirft, wobei in dieser Formel $R_6$ ein organischer Rest, bevorzugt ein Kohlenwasserstoffrest, und $R_7$ Wasserstoff oder ebenfalls ein organischer Rest ist, der mit $R_6$ gleich oder von ihm verschieden sein kann oder auch mit $R_6$ und dem Bor gemeinsam ein Ringsystem bildet. Bevorzugte organische Reste sind Alkyl-, Cycloalkyl- und/oder Arylreste. Solche das Bor substituierenden Kohlenwasserstoffreste weisen bevorzugt jeweils nicht mehr als 25 C-Atome auf. Insbesondere enthält jeder dieser Reste nicht mehr als etwa 12 bis 15 C-Atome. In einer besonders bevorzugten Ausführungsform bilden $R_6$ und $R_7$ zusammen mit dem Boratom ein Ringsystem, das die angegebenen Zahlenwerte für die Anzahl der Kohlenstoffatome nicht überschreitet. Auch hier kann das eingangs genannte 9-BBN aus praktischen Gründen bevorzugt sein.

Entscheidende Bedeutung kommt der als Matrix dienenden Esterbasis zu. Das Ausgangsmaterial für diese Matrix kennzeichnet sich dadurch, daß monofunktionelle Fettsäuren und/oder monofunktionelle Fettalkohole zu Estern beziehungsweise Estergemischen umgesetzt sind, die wenigstens in einem Anteil ihrer Fettsäure- und/oder Fettalkohol-Bestandteile additionsbereite Kohlenstoffdoppelbindungen aufweisen.

Der Begriff der Fettsäure beziehungsweise des Fettalkohols erfaßt dabei monofunktionelle Komponenten der genannten Art mit einer Kohlenstoffzahl im Bereich von etwa 8 bis 32 C-Atomen vorzugsweise im Bereich von etwa 14 bis 22 C-Atomen. Die ungesättigten Fettsäuren beziehungsweise ungesättigten Fettalkohole können synthetischen oder natürlichen Ursprungs sein. Bevorzugt handeltes sich um einoder mehrfach olefinisch ungesättigte Alkenmonocarbonsäuren beziehungsweise Monoalkenole der angegebenen Kohlenstoffzahl. Die Kohlenstoffketten dieser Fettsäuren beziehungsweise Fettalkohole können geradkettig und/oder verzweigt sein.

Die den jeweiligen Ester bildende Gegenkomponente kann ein einwertiger oder ein mehrwertiger Alkohol beziehungsweise eine monofunktionelle oder eine polyfunktionelle Carbonsäure sein. Es ist möglich, daß additionsbereite Kohlenstoff-Doppelbindungen in nur einem Bestandteil - also nur in der Fettsäure beziehungsweise in dem Fettalkohol - vorliegen, ebenso können aber auch beide esterbildenden Komponenten olefinische Doppelbindungen enthalten. Zur Herstellung der Boralkylverbindungen wird wenigstens ein substantieller Anteil dieser Doppelbindungen der Hydroborierung unterworfen.

In einer bevorzugten Ausführungsform ist die Estermatrix durch Veresterung einer monofunktionellen Komponente (Säure oder Alkohol) mit einer mehrfunktionellen Gegenkomponente (Alkohol- beziehungsweise weise Säure) gebildet. Besonders bevorzugte Matrixmaterialien können Ester ungesättigter Monocarbonsäuren (ungesättigte Fettsäuren) mit mehrwertigen Alkoholen sein. Als mehrwertige esterbildende Reaktionskomponenten sind insbesondere entsprechende Verbindungen einer Funktionalität bis zu 6 vorzugsweise mit einer Funktionalität von 2 bis 4 geeignet. So sind in der bevorzugten Ausführungsform als

Matrix für die borhaltigen Substituenten Monocarbonsäuren des angegebenen C-Zahlbereichs mit mehrwertigen Alkoholen - insbesondere mit zweiwertigen, dreiwertigen oder vierwertigen Alkoholen - verestert.

Es kann dabei zweckmäßig sein, daß die polyfunktionelle Esterkomponente eine vergleichsweise niedrige Anzahl von Kohlenstoffatomen besitzt, die beispielsweise im Bereich von 2 bis 10 vorzugsweise im Bereich von 2 bis 6 C-Atomen liegen kann. Als polyfunktionelle Alkohole eignen sich dem entsprechend besonders die niederen Glycole wie Ethylenglycol, Propylenglycol-1,2, Propylenglycol-1,3, die $C_4$-Glycole mit endständigen und/oder innenständigen Hydroxylgruppen beziehungsweise entsprechende $C_5$- und $C_6$-Verbindungen. Ein besonders bevorzugter Alkohol ist weiterhin das Glycerin oder mehrwertige Alkohole von der Art des Pentaerythrits. Umgekehrt können monofunktionelle Fettalkohole mit niederen Polycarbonsäuren insbesondere niederen Dicarbonsäuren oder niederen Tricarbonsäuren verestert sein.

Möglich ist auch die Verwendung synthetischer oder natürlicher Fette und/oder Öle als Estermatrix für die anschließende Hydroborierung. Grundsätzlich können die ungesättigten Ester in Abmischung mit gesättigten Komponenten beispielsweise in Abmischung mit gesättigten Estern und/oder als Mischung verschiedenartiger ungesättigter Ester vorliegen.

Die der Hydroborierung zugänglichen ethylenische Doppelbindungen aufweisenden Ester beziehungsweise Fette und/oder Öle können je nach Struktur und Molekulargewicht niedrig viskos fließfähig bis fest sein.

Die ungesättigten Ester beziehungsweise Estergemische wie Fette, Öle und dergleichen können vor der Hydroborierung in beliebig starkem Ausmaß ehtylenisch ungesättigt sein. Bevorzugt sind entsprechende Ausgangsmaterialien, die eine Jodzahl bis etwa 280, vorzugsweise im Bereich von etwa 1 bis 205 aufweisen. Innerhalb dieses Bereiches sind Werte der Jodzahl von etwa 5 bis 130 besonders bevorzugt.

Das Ausmaß der Hydroborierung an der Estermatrix kann im Rahmen der insgesamt vorliegenden Doppel bindungen frei gewählt werden. Es hat sich allerdings als vorteilhaft erwiesen, wenn wenigstens ein substantieller Anteil dieser Doppelbindungen durch Einführung der borhaltigen Substituenten umgesetzt wird. So sind in bevorzugten Ausführungsformen mehr als 30 %, vorzugsweise wenigstens 50 % und insbesondere wenigstens etwa 70 % der in der Estermatrix ursprünglich vorhandenen ethylenischen Doppelbindungen hydroboriert. Besonders geeignet sind solche Organoborverbindungen, in denen bezogen auf den Reaktionsansatz - wenigstens 80 %, vorzugsweise wenigstens 90 % oder sogar wenigstens 95 % der ethylenischen Doppelbindungen der Umsetzung mit den borhaltigen Komponenten zugeführt worden sind.

Zur Hydroborierung werden die ungesättigten Ester unter vollkommenem Sauerstoffausschluß durch Reaktion mit den ausgewählten Borhydridverbindungen umgesetzt. Zweckmäßig wird dabei in Lösungsmitteln gearbeitet. Geeignet sind die bekannten Lösungsmittel für Organoborverbindungen, insbesondere Tetrahydrofuran, Glycol oder Polyether wie Diethylenglycol-dimethylether, aber auch Ester, Halogenwasserstoffe und dergleichen. Die Umsetzung erfolgt zweckmäßigerweise im Temperaturbereich von etwa 0 bis 100° C insbesondere im Bereich von etwa Raumtemperatur bis 70° C.

**Weitere Einzelheiten zur Erfindung**

Die erfindungsgemäßen Bindemittelsysteme aus polymerisierbarer Komponente (A) und Härter bzw. Initiator-Komponente (B) können als zweikomponentige Formulierungen ausgebildet sein. Für die praktische Anwendung kann dann die Klebstoffkomponente (A) mit der Härterkomponente (B) vor der Applikation auf die zu verklebenden Teile vermischt werden. Die offene Zeit des Klebstoffs wird durch Abstimmung der Mengen der Komponenten (A) und (B) den Erfordernissen angepaßt.

Die Verarbeitung der erfindungsgemäßen Bindemittelsysteme ist aber auch nach der Art der "No-mix - Klebstoffe" oder "Acrylatklebstoffe der zweiten bzw. dritten Generation" möglich (vgl. hierzu "Adhesives Age", 1976, 21-24 sowie "Adhäsion", 1981, 156-161). Hier kann auf die getrennte Vermischung der Klebstoffkomponente und der Härterkomponente verzichtet werden.

Zunächst wird vielmehr die Initiator-Komponente in dünner Schicht auf eine oder beide der zu verklebenden Oberflächen aufgebracht, dann wird die Klebstoff-Komponente aufgetragen. Die Verklebung erfolgt durch Fixierung der Teile in der gewünschten Position.

Die zuvor unter a) bis f) aufgezählten Organoborverbindungen können dabei als Primerphase Verwendung finden oder darin eingesetzt werden. Besondere Bedeutung kommt den unter d) bis f) geschilderten Organoborsystemen in diesem Zusammenhang zu.

Insbesondere in diesem Zusammenhang kann einem Härtersystem Bedeutung zukommen, das in der älteren deutschen Patentanmeldung D 6391 (P 31 43 945.4) geschildert ist und wie folgt hergestellt werden kann:

g) In einer ersten Verfahrensstufe werden polymerisierbare Monomere mit ehtylenischen Doppelbindungen unter Ausschluß von Sauerstoff durch Zugabe von Organoborverbindungen polymerisiert. Nach Erreichen der verarbeitungstechnisch erforderlichen Eindickung wird diese Polymerisation durch Zugabe eines anionischen Inhibitors abgebrochen.

Man macht sich hier die literaturbekannte Tatsache zu nutze, daß Organoborverbindungen nicht nur nach einem radikalischen Mechanismus - unter Zutritt von Sauerstoff - polymerisationsauslösend wirken, daß vielmehr in Abwesenheit von Sauerstoff ethylenisch ungesättigte Monomere auch nach einem langsam ablaufenden anionischen Mechanismus durch Organoborverbindungen polymerisiert werden können, Dieser vergleichsweise langsam verlaufende Reaktionsschritt führt zur allmählichen Eindickung einer zunächst leicht

beweglichen Monomerenphase unter dem Einfluß der Organoborverbindungen. Ist die hinreichende Eindickung durch Polymerbildung erfolgt, kann die anionische Polymerisation abgebrochen werden. Auch hier liegt dann letztlich als Härterkomponente B eine Mischung von Organoborverbindungen und polymeren Komponenten vor.

Als ethylenisch ungesättigte Monomere sind hier Derivate von Acrylsäure und/oder Methacrylsäure besonders geeignet, insbesondere entsprechende Ester mit monofunktionellen oder polyfunktionellen Alkoholen sowie entsprechende Säureamide, die am Amidstickstoff in an sich bekannter Weise, beispielsweise mit Kohlenwasserstoffresten substituiert sein können.

Bezüglich der einzusetzenden Organoborverbindungen gelten die zuvor gemachten allgemeinen Angaben.

Als Inhibitoren bzw. Stabilisatoren gegenüber anionischer Polymerisation sind alle die Verbindungen gut geeignet, die auch zum Stabilisieren von α-Cyanacrylsäureestern brauchbar sind, beispielsweise saure Komponenten wie Phosphorsäure, Borsäure, Borsäureester und dergleichen, Sulfonsäuren oder Sultone.

Besonders geeignete Inhibitoren sind jedoch Carbonsäuren mit freier Carboxylgruppe. Als zweckmäßig erweist sich die Verwendung von olefinisch ungesättigten Carbonsäuren, die über ihre Doppelbindung einerseits mit Organoborhydridverbindungen reaktiv sind, andererseits aber auch über diese Doppelbindung in eine Polymerisationsreaktion eingehen können und damit letztlich aktiver Klebstoffbestandteil werden. Besonders geeignet als Inhibitoren gegen anionische Polymerisation sind dementsprechend Acrylsäure und/oder Methacrylsäure. Ihre freie Carboxylgruppe bringt nicht nur den anionisch verlaufenden Polymerisationsprozeß zum Stillstand, im Klebstoffsystem erfüllen die freien Carboxylgruppen in an sich bekannter Weise ihre haftverbessernde Wirkung.

Schließlich ist es erfindungsgemäß aber möglich, einkomponentige Klebstoffsysteme einzusetzen, die gemäß der Lehre der europäischen Offenlegungsschrift 0 051 796 (D 6246 EP)

aufgebaut sind, als polymerisierbare Komponente aber die eingangs genannten resorbierbaren (Meth)-acrylatverbindungen enthalten.

**a) Mengenverhältnis von Härter zu Klebstoffkomponenten**

Zur Härtung der erfindungsgemäßen Reaktionsmassen setzt man von den beschriebenen polymeren Borinitiatoren etwa 0,1 bis 40 Gewichtsprozent insbesondere etwa 0,1 bis 30 Gewichtsprozent - jeweils bezogen auf den zu polymerisierenden Anteil - ein. Bevorzugt werden die polymeren Härter in Mengen von etwas 0,5 bis 30 Gewichtsprozent - bezogen auf den zu polymerisierenden Anteil - verwendet.

**b) mitzuverwendende Polymere**

Häufig enthalten die polymerisierbaren Massen zusätzlich zu den polymerisierbaren Komponenten vorgefertigte Polymerisate wie Polymethyl(meth)acrylat, Copolymere von Methyl(meth)acrylat, Polychloropren, chlorsulfoniertes Polyethylen, Nitrilkautschuke und Urethane zur Verstärkung beziehungsweise Elastifizierung und gleichzeitig als Verdickungsmittel. Um die Abbaubarkeit der gehärteten Systeme zu gewährleisten sind Polyhydroxycarbonsäuren, wie zum Beispiel Polyglycolsäure und Polymilchsäure, besonders geeignet. Hierdurch wird die Verarbeitung der Massen, beispielsweise des Klebstoffes, erleichtert. Im einzelnen gelten die Angaben des einschlägigen Standes der Technik.

In vielen Fällen ist es zweckmäßig oder notwendig, weitere Hilfsstoffe wie Füllstoffe, beispielsweise Quarzmehl oder dergleichen, zuzugeben. Schließlich kann es zweckmäßig sein, mit geeigneten Farbstoffen beziehungsweise Pigmenten einzufärben.

**c) mitzuverwendende Comonomere**

Als zusätzliche polymerisierbare Bestandteile können in den erfindungsgemäßen Kunststoffmassen die zahlreichen bekannten Verbindungen mit polymerisierbarer ethylenischer Doppelbindung eingesetzt werden, die ublicherweise in beispielsweise Gießharzen, Füllstoffen und insbesondere in Reaktionsklebstoffen zur Verwendung kommen. Insbesondere geeignet sind dementsprechend die Ester von Acrylsäure und/oder α-substituierten Acrylsäuren, wie Methacrylsäure - im folgenden als (Meth)-acrylsäureverbindungen bezeichnetmit monovalenten oder polyvalenten insbesondere zweiwertigen Alkoholen. Geeignet sind aber auch andere bekannte Derivate der (Meth)-acrylsäure, insbesondere die entsprechenden Säureamide, die am Amidstickstoff auch zum Beispiel mit Kohlenwasserstoffresten substituiert sein können. Weitere mögliche Substituenten in -Stellung der (Meth)-acrylsäurederivate sind beispielsweise Halogen, insbesondere Chlor und/oder Brom, Cyan oder allgemein Alkylreste mit bis zu 10 C-Atomen.

Als Beispiele für (Meth)-acrylsäureester monovalenter Alkohole seien genannt: (Meth)-acrylsäuremethylester, (Meth)-acrylsäureethylester, (Meth)-acrylsäurebutylester, (Meth)-acrylsäureethylhexylester.

Beispiele entsprechender Ester mit polyvalenten Alkoholen sind solche mit Ethylenglykol, Diethylenglykol,

Polyethylenglykol und Trimethylolpropan, Diund Mono-(meth)-acrylsäureester von Glycerin, Di(meth)acrylsäureester von Tri- und Tetraethylenglykol, von Di-, Tri-, Tetra- und Pentapropylenglykol, die Di-(meth)-acrylsäureester von ethoxyliertem oder propoxyliertem Diphenylolpropan. Auch kommen (Meth)acrylsäureester von Alkoholen in Frage, die sich von Dihydroxymethyltricyclodecan ableiten oder auch solche, die auf Basis von Tricyclodecan hergestellt worden sind, wobei zwei alkoholische Funktionen im Ringsystem durch Umsetzung mit Dicarbonsäuren wie Maleinsäure oder auch Cyclohexandicarbonsäure oder Terephthalsäure verlängert sind.

Weiterhin sind verwendbar Umsetzungsprodukte des Diglycidylethers von Diphenylolpropan mit Methacrylsäure und/oder Acrylsäure. Auch sind Reaktionsprodukte von Diisocyanaten oder Triisocyanaten, zum Beispiel Toluylendiisocyanat, Diphenylmethandiisocyanat, Isophorondiisocyanat, trimerisiertem Toluylenisocyanat und dergleichen mit Hydroxyalkyl-(meth)-acrylaten als polymerisierbare Bestandteile brauchbar.

Geeignet sind aber auch polymerisierbare Monomere wie Vinylacetat, Vinylhalogenide, zum Beispiel Vinylchlorid, Vinylbromid oder Vinylfluorid, Styrol, Diphenylbenzol, Crotonsäure- und Maleinsäureester oder die sogenannten, gegebenenfalls styrolisierten ungesättigten Polyesterharze. Diese zuletzt genannten Verbindungen werden im allgemeinen in Reaktionsklebstoffen nur in untergeordneter Menge, beispielsweise in Mengen bis zu 25 Gewichtsprozent der polymerisierbaren Bestandteile, mitverwendet.

Außerdem kommen in Betracht 2-Acryloyloxyethyl-phosphat, 2-Methacryloyloxyethylphosphat, Bis-2-acryloyloxyethylphosphat, Bis-2-methacryloyloxyethyl-phosphat, Tris-2-acryloyloxyethylphosphat, Tris-2-methacryloyloxyethylphosphat und Säureamide wie etwa Dimethylenbis(meth)-acrylamid, Tetramethylenbis(meth)acrylamid, Trimethylhexamethylenbis(meth) acrylamid, Tri(meth)acryloyldiethylentriamin und dergleichen mehr.

**Beispiele**

**Probekörper**

(vergl. hierzu G. Giebel et al., Biomed. Techn. 26, 170, (1981))

Knochenprüfkörper aus Rindercortalis mit den Abmessungen 50 x 20 x 5 mm wurden in der Mitte - exakt rechtwinklig zu den Seitenflächen (50 x 5 mm) - mit einer Diamant-Trennsäge unter Wasserspülung osteotomiert.

Auf diese Weise wurden paßgenaue Fügeteile mit einer Berührungsfläche von 1 $cm^2$ hergestellt.

**Probekörpervorbehandlung**

(vergl. Giebel et al., aaO)

Die nicht weiter vorbehandelten Knochenprüfkörper wurden 2 Stunden in eine 37° C warme Ringerlösung gelegt, um die Knochen intensiv zu benetzen.

**Verklebung**

a) 2-K-Klebstoffe

Klebstoff harz (A) und Härter (B) werden gemischt. Das Gemisch wird auf den feuchten Knochen aufgetragen.

b) No-mix-Klebstoffe

Nur der Härter (A) wird in dünner Schicht auf beide zu verklebenden Flächen aufgetragen. Direkt am Anschluß wird das Klebstoffharz (B) appliziert.

Die Prüfkörper werden gefügt, in eine Presse aus PVC überführt und mit 5 N belastet. Die Klebepresse verhindert ein seitliches Verschieben der Prüfkörper während der Aushärtung.

**Aushärtebedingungen**

(vergl. Giebel et al aaO)

Die gefügten Knochen werden in der Klebepresse 20 min lang in einer Klimakammer bei 37° C und ca. 100 % relativer Luftfeuchtigkeit gelagert. Im Anschluß werden sie der Klebepresse entnommen und 20 Std. in Ringerlösung bei 37° C gelagert.

**Festigkeitsmessung**

Die Zugfestigkeit wird an einer Universalprüfmaschine mit einer Vorschubgeschwindigkeit von 12 mm/min zerrissen.

Aufgrund der durch das natürliche Substrat vorgegebenen breiten Steuung der Messwerte wurden jeweils die Festigkeitsbereiche aus 6 Einzelversuchen angegeben.

**Harzkomponente A**

**Beispiele 1 bis 6**

**Oligohydroxycarbonsäuren mit Hydroxylendgruppen**

a) Darstellung aus Glycolsäure und Ethylenglycol

In einem Dreihalskolben mit Rührer und Destillationsbrücke werden Glycolsäure und Ethylenglycol vorgelegt. Unter Stickstoff wird schnell auf 150°C und dann im Verlauf von 6 Stunden von 150 auf 200° C hochgeheizt. Dabei spaltet sich bereits der größte Anteil des Reaktionswassers, der den Umsatz der Esterkondensation anzeigt, ab. Man läßt den Ansatz auf etwa 150° C abkühlen, evakuiert vorsichtig auf 10 Torr und vervollständigt den Umsatz bei 200°C und 10 Torr. Nach 30 Minuten wird das Frodukt bei ca. 150° C heiß abgefüllt. Die Zusammensetzung der Ansätze und die Oligomereigenschaften sind der Tabelle 1 (Beispiele 1 bis 3) zu entnehmen.

**Tabelle 1**

Oligohydroxycarbonsäuren mit Hydroxylendgruppen aus Glycolsäure und Ethylenglycol

| Beispiel | Edukte<br>Glycolsäure<br>mol | <br>Ethylenglycol<br>mol | Reaktionswasser<br>% d. Th. | Säurezahl | berechnetes Molgewicht<br>g / mol | Beschaffenheit |
|---|---|---|---|---|---|---|
| 1 | 3 | 1 | 100 | 14 | 252 | klar, viskos, hellgelb |
| 2 | 4 | 1 | >90 | 20 | 294 | viskos, weiß |
| 3 | 6 | 1 | >98 | 24 | 410 | wachsartig, weiß |

b) Darstellung aus Milchsäureethylester und Ethylenglycol

In einem Dreihalskolben mit Rührer und Destillationsbrücke werden Milchsäureethylester und Ethylenglycol vereinigt. Als Katalysator werden 70 ml 0,2%ige methanolische Natriummethylatlösung zugegeben und der Ansatz im Anschluß auf 150° C erhitzt. Man steigert die Temperatur vorsichtig weiter bis auf 180°C,so daß bei ca. 80° C konstant Ethanol abdestilliert. Nach dem Ende der Ethanolabspaltung nach ca. 16 Stunden wird bei 150° C Badtemperatur auf 10 Torr evakuiert und die Substanz im Anschluß unter Stickstoff abgefüllt. Die Zusammensetzung der Ansätze und die Oligomereigenschaften sind der Tabelle 2 (Beispiele 4 bis 6) zu entnehmen.

**Tabelle 2**

Oligohydroxycarbonsäuren mit Hydroxylendgruppen aus Milchsäureethylester und Ethylenglycol

| Beispiel | Edukte | | Produkte | | |
|---|---|---|---|---|---|
| | Milchsäureethylenster mol | Ethylenglycol mol | Ausbeute Ethanol % | Molgewicht | Beschaffenheit |
| 4 | 2 | 1 | 95 | 193 (Osmose) | klar, viskos, orangenfarben |
| 5 | 4 | 1 | 92 | | klar, viskos, braun |
| 6 | 6 | 1 | 96 | | klar, viskos, rotbraun |

**Beispiele 7 bis 10**

**Olihydroxycarbonsäuren mit Carboxylendgruppen**

Allgemeine Vorschrift

a) Durch Umsetzung von hydroxylgruppenterminierten Oligohydroxycarbonsäuren mit Bernsteinsäureanhydrid

**Beispiel 7**

206,3 g des Oligomeren aus Beispiel 4, 200 g Bernsteinsäureanhydrid und 1 g Benzyltrimethylammoniummethoxid (40%ig in Methanol) werden in einem Dreihalskolben mit Rührer, Rückflußkühler und Stickstoffeinleitung vereinigt und 8 Stunden bei 80° C unter Stickstoff gerührt. Das Produkt ist durch folgende Kennzahlen charakterisiert:

Säurezahl: 296

OH-Zahl: <3

b) Darstellung aus Glycolsäure und Adipinsäure

In einem Dreihalskolben mit Rührer und Destillationsbrücke werden Dicarbonsäure und Hydroxycarbonsäure vorgelegt. Unter Stickstoff wird schnell auf 150° C und dann im Verlauf von 6 Stunden von 150 auf 200° C hochgeheizt. Dabei spaltet sich bereits der größte Teil des Reaktionswassers, der den Umsatz der Esterkondensation anzeigt, ab. Man läßt den Ansatz auf etwa 150° C abkühlen, evakuiert vorsichtig auf 10 Torr und vervollständigt den Umsatz bei 200° C und 10 Torr. Das Produkt wird heiß unter Stickstoff abgefüllt. Die Zusammensetzung der Ansäzte und die Oligomereigenschaften sind der Tabelle 3 (Beispiele 8 bis 10) zu entnehmen.

**Tabelle 3**

Oligohydroxycarbonsäuren mit Carboxylendgruppen aus Glycolsäure und Adipinsäure

| Beispiel | Edukte | | Produkte | | | |
|---|---|---|---|---|---|---|
| | Glycolsäure mol | Adipinsäure mol | Ausbeute Reaktionswasser % | Säurezahl | Molgewicht aus Säurezahl $g\ mol^{-1}$ | Beschaffenheit |
| 8 | 1 | 1 | > 99 | 620 | 181 | wachsartig, fest |
| 9 | 3 | 1 | > 98 | 376 | 298 | wachsartig, weich |
| 10 | 4 | 1 | > 96 | 334 | 336 | wachsartig, weich |

**Bespiele 11 bis 20**

**Oligohydroxycarbonsäuren mit polymerisierbaren Endgruppen**

a) Oligohydroxycarbonsäuren mit polymerisierbaren Endgruppen aus Oligohydroxycarbonsäuren mit endständigen Hydroxylgruppen

In einem Dreihalskolben mit Rührer und Wässerabscheider werden die Oligohydroxycarbonsäuren mit Hydroxylendgruppen und 1,1 Äquivalent Methacrylsäure pro Hydroxylgruppe vorgelegt. Gleichzeitig werden - bezogen auf Methacrylsäure - gleiche Gewichtsteile Toluol und jeweils 2 Gewichtsprozent p-Toluolsulfonsäure und Hydrochinon zugegeben.

Das Gemisch wird unter schnellem Rühren zum Sieden erhitzt und das gebildete Reaktionswasser am Wasserabscheider abgetrennt.

Wenn sich nach 5 Stunden Reaktionszeit weniger als 90 bis 95 Gewichtsprozent des theoretisch zu erwartenden Reaktionswassers abgespalten haben, werden nochmals 20 % der ursprünglich zugegebenen Menge Methacrylsäure mit jeweils 2 Gewichtsprozent para-Toluolsulfonsäure und Hydrochinon zugegeben und die Reaktion weitergeführt.

Die Reaktion ist beendet, sobald sich 90 bis 95 % des zu erwartenden Reaktionswassers gebildet haben. Das Reaktionsprodukt wird nach dem Erkalten in das doppelte Volumen Ethanol gegeben und filtriert. Die klare ethanolische Lösung engt man am Rotationsverdampfer auf 1/4 des ursprünglichen Volumens ein, gießt sie in die gleiche Menge Wasser und neutralisiert sie mit Natriumhydrogencarbonat. Die organische Phase wird abgetrennt, die wäßrige Phase mit Toluol ausgeschüttelt die organischen Phasen vereinigt, dreimal mit Wasser gewaschen und über Natriumsulfat getrocknet. Das Lösungsmittel wird bei Raumtemperatur zunächst am Rotationsverdampfer, dann bei $10^{-4}$ Torr abgezogen. Die Zusammensetzung der Ansätze und die Eigenschaften der polymerisierbaren Oligomeren sind in der Tabelle 4 (Beispiele 11 bis 16) aufgeführt.

**Tabelle 4**

Oligohydroxycarbonsäuren mit polymerisierbaren Endgruppen

| Beispiel | Edukt Diol aus Beispiel | Ausbeute Reaktions-wasser % | Beschaffenheit |
|---|---|---|---|
| 11 | 1 | > 97 | homogen, dünnflüssig, braun |
| 12 | 2 | > 95 | homogen, dünnflüssig, braun |
| 13 | 3 | > 95 | homogen, dünnflüssig, braun |
| 14 | 4 | > 95 | homogen, dünnflüssig, braun |
| 15 | 5 | > 95 | homogen, viskos, braun |
| 16 | 6 | > 90 | homogen, viskos, braun |

**b) Oligohydroxycarbonsäuren mit polymerisierbaren Endgruppen aus Oligohydroxycarbonsäuren mit endständigen Carboxylgruppen**

In einem Dreihalskolben mit Rührer und Rückflußkühler wird die Oligohydroxycarbonsäure mit endständigen Carboxylgruppen vorgelegt und 1,0 Äquivalent Glycidylmethacrylat mit 0,1 Gewichtsprozent Benzyltrimethylammoniummethoxid und 0,06 Gewichtsprozent Hydrochinon zugegeben. Unter Rühren wird das Gemisch innerhalb von 45 Minuten auf 80° C erhitzt und die Reaktion bei 80°C so lange weitergeführt, bis die Säurezahl unter 35 abgesunken ist. Die Reaktion ist üblicherweise nach 10 bis 20 Stunden beendet.

Die Zusammensetzung der Ansätze und die Eigenschaften der polymerisierbaren Oligomeren sind in der Tabelle 5 (Beispiele 17 bis 20) aufgeführt.

**Tabelle 5**

Oligohydroxycarbon säuren mit polymerisierbaren Endgruppen

| Beispiel | Edukt<br>Dicarbonsäure aus Beispiel | Produkt<br>Säurezahl | <br>Beschaffenheit |
|---|---|---|---|
| 17 | 7 | 24 | homogen, viskos, gelb |
| 18 | 8 | 30 | homogen, viskos, hell-gelb |
| 19 | 9 | 34 | homogen, hochviskos, hellgelb |
| 20 | 10 | 31 | homogen, hochviskos, hellgelb |

**Initiatorkomponenten B**

**Initiatorkomponente B 1**

In einem Dreihalskolben mit Rührer und Destillationsbrücke werden 4 Mol Trimethyladipinsäure und 5 Mol Hexamethylenglycol vorgelegt. Unter Stickstoff wird schnell auf 150° C und dann im Verlauf von 6 Stunden von 150 auf 200° C hochgeheizt. Dabei spaltet sich bereits der größte Anteil des Reaktionswassers, der den Umsatz der Esterkondensation anzeigt ab. Man läßt den Ansatz auf etwa 150° C abkühlen, evakuiert vorsichtig auf 10 Tor und vervollständigt den Umsatz bei 200° C und 10 Torr. Der Oligoester wird heiß abgefüllt. Er ist viskos und braur gefärbt.

Zur Befreiung von Restsauerstoff werden 100 g des Oligoesters in 100 g frischdestilliertem Tetrahydrofuran gelöst. Im Anschluß wird das Lösungsmittel im Vakuum bei $10^{-4}$ Torr abgezogen. In einer Glovebox werden zu 100 g des Oligoesters erneut 100 g destilliertes, entgastes THF zugegeben und in diese Lösung unter vollständigem Sauerstoffausschluß 41 g 9-Borabicyclo [3.3.1]nonan eingetragen. Das Gemisch wird zur weiteren Homogenisierung gerührt. Im Anschluß erhitzt man 1 Stunde unter Rühren auf 60° C. Das THF wird im Vakuum abgezogen und das Vorratsgefäß geschlossen. Die Entnahme von Proben erfolgt unter Schutzgas und vollständigem Sauerstoffausschluß. Das Produkt ist eine viskose, dunkelbraune Flüssigkeit.

**Initiatorkomponente B 2**

5,0 g 9-BBN werden unter vollständigem Sauerstoffausschluß in einem 250ml-Kolben vorgelegt. In diesen Kolben werden im Hochvakuum ($10^{-4}$ Torr) 100 g trockenes, frisch destilliertes Methylmethacrylat umkondensiert. Der Kolben wird verschlossen und geschüttelt, bis das 9-BBN in Lösung gegangen ist. Nach 2 Tagen zeigt das Gemisch eine deutlich erhöhte Viskosität. Es werden dann - ebenfalls unter vollständigem Sauerstoffausschluß - 0,5 g sauerstofffreie, unter Argon destillierte Methacrylsäure und 5,0 g 9-BBN zugegeben. Diese Mischung ist ohne weitere Viskositätszunahme lagerstabil.

**Initiatorkomponenten B 3 bis B 4**

Zur Befreiung von Restsauerstoff werden die in der Tabelle 6 aufgelisteten Fette, Öle oder Fettsäurederivate in der gleichen Menge THF gelöst. Im Anschluß wird das Lösungsmittel im Vakuum bei $10^{-4}$ Torr abgezogen. In einer Glovebox werden erneut gleiche Gewichtsteile frischdestilliertes, entgastes THF zugegeben und die Fette, Öle oder Fettsäurederivate gelöst. Unter vollständigem Sauerstoffausschluß werden die in der Tabelle 6 aufgelisteten Mengen an 9-Borabi-cyclo [3.3.1] nonan (9-BBN) zugegeben und das Gemisch so lange gerührt, bis das 9-BBN quantitativ in Lösung gegangen ist. Im Anschluß erhitzt man 1 Stunde unter Rühren auf 60° C. Das THF wird im Vakuum abgezogen und das Vorratsgefäß verschlossen. Die Entnahme von Proben erfolgt unter Schutzgas und vollständigem Sauerstoffausschluß.

**Tabelle 6**

**Darstellung der Initiatorkomponenten B 3 - B 4**

| Nr. | Öl, Fett, Fettsäurederivat | Jod-zahl | eingesetzte Menge 9-BBN/g pro 100 g Substanz | mol-% 9-BBN bezogen auf Doppelbindungen | Produkteigenschaften |
|---|---|---|---|---|---|
| B 3 | Ölsäuremethylester 1) | 85,8 | 41 | 100 | homogen, niedrigviskos, gleb |
| B 4 | Leinöl | 178 | 86 | 100 | homogen, viskos, hellgelb |

1) Der Umsatz der Reaktion wurde anhand des $^{1}$H-NMR-Spectrums (-C$\underline{H}$ = C$\underline{H}$-Resonanz bei =5,3 ppm) und des $^{13}$C-NMR-Spectrums (-C$\underline{H}$ = C$\underline{H}$-Resonanzen bei = 129,5 und 129,8 ppm) verfolgt.
Der Umsatz an der Doppelbindung beträgt im Beispiel B 3 70 ± 5 %.

**Ergebnisse**

Ausgewählte Monomere wurden mit jeweils 5 Gewichtsprozent eines Gemisches aus Methacryloyloxyethylphosphat und Bismethacryloyloxyethylphosphat gemischt und als Harzkomponente (A) eingesetzt. Bei der Verklebung von Knochenmaterial unter "simulierten in-vivo-Bedingungen" wurden mit den angegebenen Härtern (B) (jeweils 1 bis 3 Gewichtsprozent) folgende Zugfestigkeiten erzielt (Tabelle 7).

**Tabelle 7**

**Übersicht über die gemessenen Zugfestigkeiten /Ncm$^{-2}$**

| Harzkomponente A | Härterkomponente B | | | |
|---|---|---|---|---|
| | B 1 (2K) | B 2 (no-mix) | B 3 (2K) | B 4 (2K) |
| Beispiel 12 | 430 - 1070 | 90 - 140 | | |
| Beispiel 13 | 260 - 660 | 150 - 550 | | 240 - 410 |
| Beispiel 18 | 180 - 400 | 180 - 330 | 90 - 200 | 130 - 200 |

**Patentansprüche** für die Vertragsstaaten BE CH DE FR GB IT LI NL SE

1. Zum Verkleben von körpereigenem Hartgewebe geeignetes chirurgisches Bindemittelsystem auf Basis eines polymerisierbaren (Meth)acrylatklebers und eines polymerisationsauslösenden Starters, dadurch gekennzeichnet, daß es als resorbierbare (Meth)-acrylatkomponente bei Raumtemperatur flüssige bis feste (Meth) - acrylsäureester mit (Meth)acrylatresten an Polyester-Oligomerketten aus Hydroxycarbonsäuren und als Starter Organoborverbindungen enthält.

2. Bindemittel nach Anspruch 1, dadurch gekennzeichnet, daß die resorbierbare (Meth)-acrylatkomponente wenigstens anteilsweise mehr als einen (Meth)-acrylatrest - vorzugsweise 2 (Meth)-acrylatreste - an den Hydroxycarbonsäure-Polyesteroligomeren aufweist.

3. Bindemittel nach Ansprüchen 1 und 2, dadurch gekennzeichnet, daß die (Meth)-acrylatreste wenigtens anteilsweise endständig an den Polyesteroligomeren vorliegen, wobei bevorzugt 2 (Meth)-acrylatreste in $\alpha$, $\omega$ - Stellung vorgesehen sind.

4. Bindemittel nach Ansprüchen 1 bis 3, dadurch gekennzeichnet, daß die Polyester-Oligomeren aus Monohydroxy-Monocarbonsäuren gebildet sind, die vorzugsweise 2 bis 10, insbesondere 2 bis 6 C-Atome, aufweisen.

5. Bindemittel nach Ansprüchen 1 bis 4, dadurch gekennzeichnet, daß die Polyester-Oligomeren aus Glycolsäure, Milchsäure, Hydroxypropionsäure, Hydroxybuttersäure und/oder Hydroxybenzoesäure aufgebaut sind.

6. Bindemittel nach Ansprüchen 1 bis 5, dadurch gekennzeichnet, daß die Polyester-Oligomeren ein mittleres Molekulargewicht im Bereich von etwa 200 bis 600, vorzugsweise im Bereich von etwa 300 bis 500, besitzen.

7. Bindemittel nach Ansprüchen 1 bis 6, dadurch gekennzeichnet, daß die Polyester-Oligomeren unter Mitverwendung von monofunktionellen und/ oder vorzugsweise difunktionellen Alkoholen oder Carbonsäuren bzw. Carbonsäureanhydriden und/oder unter Mitverwendung von primären, sekundären und/oder tertiären Monoaminen hergestellt worden sind.

8. Bindemittel nach Ansprüchen 1 bis 7, dadurch gekennzeichnet, daß bei der Herstellung der Polyester-Oligomeren die gegebenenfalls eingesetzten monofunktionellen Komponenten in Mengen nicht über 95 Mol-Prozent, vorzugsweise nicht über 50 Mol-Prozent, und insbesondere in Mengen nicht über 10 Mol-Prozent - jeweils bezogen auf die Mischung der monofunktionellen Komponenten und der Dialkohole bzw. Dicarbonsäuren - verwendet worden sind.

9. Bindemittel nach Ansprüchen 1 bis 8, dadurch gekennzeichnet, daß die resorbierbaren (Meth)acrylatkomponenten bei Raumtemperatur fließfähig bis pastös verstreichbar sind und dabei bevorzugt bei Raumtemperatur eine Viskosität im Bereich von etwa 500 bis 70.000 mPas aufweisen.

10. Bindemittel nach Ansprüchen 1 bis 9, dadurch gekennzeichnet, daß sie Organoborverbindungen als Starter enthalten, die an Luft praktisch keine Selbstentzündlichkeit besitzen, gleichzeitig aber bei Körpertemperatur unter Luftzutritt zur Polymerisationsauslösung befähigt sind.

11. Bindemittel nach Ansprüchen 1 bis 10, dadurch gekennzeichnet, daß sie als Starter Alkyl- und/oder Arylreste aufweisende Organoborverbindungen enthalten, die auch B-H-Bindungen besitzen können.

12. Bindemittel nach Ansprüchen 1 bis 11, dadurch gekennzeichnet, daß sie als Starter wenigstens eine Bortrialkylverbindung und/oder wenigstens eine Boralkylhydridverbindung - in diesem Fall insbesondere ein Dialkylborhydrid - enthalten.

13. Bindemittel nach Ansprüchen 1 bis 12, dadurch gekennzeichnet, daß sie als 2-Komponenten-Systeme vorliegen, deren Komponenten - polymerisierbare Komponente (A) und Starter enthaltende Komponente (B) - bei der Anwendung miteinander vermischt oder auch nach dem No-mix-Verfahren verarbeitet werden.

14. Bindemittel nach Ansprüchen 1 bis 13, dadurch gekennzeichnet, daß als Starterkomponente (B) wenigstens eines der folgenden Systeme vorliegt:

a) eine homogene Mischung wenigstens einer durch Luftzutritt aktivierbaren Organoborverbindung mit wenigstens einem bei Raumtemperatur flüssigen bis festen, vorzugsweise mit der Komponente (A) verträglichen, den Organoborverbindungen gegenüber inerten organischen Oligomeren oder Polymeren;

b) polymere Organoborverbindungen, die als Substituenten an einer gegen Luftzutritt stabilen Polymermatrix Borwasserstoffund/oder Organoborreste aufweisen;

c) Boralkylverbindungen, die Borwasserstoffreste und/oder Organoborreste an Fettsäure- und/ oder Fettalkohol-Estern enthalten.

15. Bindemittel nach Anspruch 14, dadurch gekennzeichnet, daß das Oligomere oder Polymere gemäß a) ein Polymerisat auf Basis von Derivaten - vorzugsweise Estern und/oder gegebenenfalls substituierten Säureamiden - der (Meth)-acrylsäure ist, das insbesondere unter Ausschluß von Sauerstoff in situ unter dem Einfluß der Organoborverbindungen aus seinen Monomeren hergestellt worden ist.

16. Verwendung der Bindemittelsysteme nach Ansprüchen 1 bis 15 zur Herstellung von resorbierbaren Formteilen bei der Verklebung von körpereigenem Hartgewebe gegebenenfalls zusammen mit Kunststoff und/oder Metall.

**Patentansprüche** für den Vertragsstaat AT

1. Verfahren zur Herstellung eines zum Verkleben von körpereigenem Hartgewebe geeigneten chirurgischen Bindemittelsystems auf Basis eines polymerisierbaren (Meth)acrylatklebers und eines polymerisationsauslösenden Starters, dadurch gekennzeichnet, daß man als resorbierbare (Meth)-acrylatkomponente bei Raumtemperatur flüssige bis feste (Meth)-acrylsäureester mit (Meth)acrylatresten an Polyester-Oligomerketten aus Hydroxycarbonsäuren und als Starter Organoborverbindungen einsetzt.

2. Verfahren nach Anspruch 1, dadurch gekennzeichnet, daß die resorbierbare (Meth)-acrylatkomponente wenigstens anteilsweise mehr als einen (Meth)-acrylatrest - vorzugsweise 2 (Meth)-acrylatreste - an den Hydroxycarbonsäure-Polyesteroligomeren aufweist.

3. Verfahren nach Ansprüchen 1 und 2, dadurch gekennzeichnet, daß die (Meth)-acrylatreste wenigstens anteilsweise endständig an den Polyesteroligomeren vorliegen, wobei bevorzugt 2 (Meth)-acrylatreste in $\alpha,\omega$ - Stellung vorgesehen sind.

4. Verfahren nach Ansprüchen 1 bis 3, dadurch gekennzeichnet, daß die Polyester-Oligomeren aus Monohydroxy-Monocarbonsäuren gebildet sind, die vorzugsweise 2 bis 10, insbesondere 2 bis 6 C-Atome, aufweisen.

5. Verfahren nach Ansprüchen 1 bis 4, dadurch gekennzeichnet, daß die Polyester-Oligomeren aus Olycolsäure, Milchsäure, Hydroxypropionsäure, Hydroxybuttersäure und/oder Hydroxybenzoesäure aufgebaut sind.

6. Verfahren nach Ansprüchen 1 bis 5, dadurch gekennzeichnet, daß die Polyester-Oligomeren ein mittleres Molekulargewicht im Bereich von etwa 200 bis 600, vorzugsweise im Bereich von etwa 300 bis 500, besitzen.

7. Verfahren nach Ansprüchen 1 bis 6, dadurch gekennzeichnet, daß die Polyester-Oligomeren unter Mitverwendung von monofunktionellen und/oder vorzugsweise difunktionellen Alkoholen oder Carbonsäuren bzw. Carbonsäureanhydriden und/oder unter Mitverwendung von primären, sekundären und/oder tertiären Monoaminen hergestellt worden sind.

8. Verfahren nach Ansprüchen 1 bis 7, dadurch gekennzeichnet, daß bei der Herstellung der Polyester-Oligomeren die gegebenenfalls eingesetzten monofunktionellen Komponenten in Mengen nicht über 95 Mol-Prozent, vorzugsweise nicht über 50 Mol-Prozent, und insbesondere in Mengen nicht über 10 Mol-Prozent - jeweils bezogen auf die Mischung der monofunktionellen Komponenten und der Dialkohole bzw. Dicarbonsäuren - verwendet worden sind.

9. Verfahren nach Ansprüchen 1 bis 8, dadurch gekennzeichnet, daß die eingesetzten resorbierbaren (Meth)acrylatkomponenten bei Raumtemperatur fließfähig bis pastös verstreichbar sind und dabei bevorzugt bei Raumtemperatur eine Viskosität im Bereich von etwa 500 bis 70.000 mPas aufweisen.

10. Verfahren nach Ansprüchen 1 bis 9, dadurch gekennzeichnet, daß man Organoborverbindungen als Starter einsetzt, die an Luft praktisch keine Selbstentzündlichkeit besitzen, gleichzeitig aber bei Körpertemperatur unter Luftzutritt zur Polymerisationsauslösung befähigt sind.

11. Verfahren nach Ansprüchen 1 bis 10, dadurch gekennzeichnet, daß man als Starter Alkyl- und/oder Arylreste aufweisende Organoborverbindungen einsetzt, die auch B-H-Bindungen besitzen können.

12. Verfahren nach Ansprüchen 1 bis 11, dadurch gekennzeichnet, daß man als Starter wenigstens eine Bortrialkylverbindung und/oder wenigstens eine Boralkylhydridverbindung - in diesem Fall insbesondere ein Dialkylborhydrid - einsetzt.

13. Verfahren nach Ansprüchen 1 bis 12, dadurch gekennzeichnet, daß man ein 2-Komponenten-System herstellt und dessen Komponenten - polymerisierbare Komponente (A) und Starter enthaltende Komponente (B) - bei der Anwendung miteinander vermischt oder auch nach dem No-mix-Verfahren verarbeitet.

14. Verfahren nach Ansprüchen 1 bis 13, dadurch gekennzeichnet, daß man als Starterkomponente (B) wenigstens eines der folgenden Systeme einsetzt:

a) eine homogene Mischung wenigstens einer durch Luftzutritt aktivierbaren Organoborverbindung mit wenigstens einem bei Raumtemperatur flüssigen bis festen, vorzugsweise mit der Komponente (A) verträglichen, den Organoborverbindungen gegenüber inerten organischen Oligomeren oder Polymeren;

b) polymere Organoborverbindungen, die als Substituenten an einer gegen Luftzutritt stabilen Polymermatrix Borwasserstoff— und/oder Organoborreste aufweisen;

c) Boralkylverbindungen, die Borwasserstoffreste und/oder Organoborreste an Fettsäure- und/ oder Fettalkohol-Estern enthalten.

15. Verfahren nach Anspruch 14, dadurch gekennzeichnet, daß man als Oligomere oder Polymere gemäß a) ein Polymerisat auf Basis von Derivaten - vorzugsweise Estern und/oder gegebenenfalls substituierten Säureamiden - der (Meth)-acrylsäure einsetzt, das insbesondere unter Ausschluß von Sauerstoff in situ unter dem Einfluß der Organoborverbindungen aus seinen Monomeren hergestellt worden ist.

16. Verwendung der nach Ansprüchen 1 bis 15 hergestellten Bindemittelsysteme zur Herstellung von resorbierbaren Formteilen bei der Verklebung von körpereigenem Hartgewebe gegebenenfalls zusammen mit Kunststoff und/oder Metall.

**Claims** for the Contracting States BE CH DE FR GB IT LI NL SE

1. A surgical binder system suitable for bonding hard body tissue based on a polymerizable (meth)acrylate adhesive and a polymerization-initiating starter, characterized in that it contains (meth)acrylates — liquid to solid at room temperature - with (meth)acrylate residues on polyester-oligomer chains of hydroxycarboxylic acids as the resorbable (meth)acrylate component and an organoboron compound as starter.

2. A binder as claimed in Claim 1, characterized in that the resorbable (meth)acrylate component contains at least proportionally more than one (meth)acrylate residue, preferably two (meth)acrylate residues, on the hydroxycarboxylic acid polyester oligomers.

3. A binder as claimed in Claims 1 and 2, characterized in that the (meth)acrylate residues are at least proportionally present at the ends of the polyester oligomer chains, preferably two (meth)acrylate residues being provided in the $\alpha,\omega$ -position.

4. A binder as claimed in Claims 1 to 3, characterized in that the polyester oligomers are formed from monohydroxy monocarboxylic acids containing preferably 2 to 10 and, more particularly, 2 to 6 carbon atoms.

5. A binder as claimed in Claims 1 to 4, characterized in that the polyester oligomers are synthesized from glycolic acid, lactic acid, hydroxypropionic acid, hydroxybutyric acid and/or hydroxybenzoic acid.

6. A binder as claimed in Claims 1 to 5, characterized in that the polyester oligomers have an average molecular weight of from about 200 to 600 and preferably of from about 300 to 500.

7. A binder as claimed in Claims 1 to 6, characterized in that the polyester oligomers have been produced using monofunctional and/or preferably difunctional alcohols or carboxylic acids or carboxylic acid anhydrides and/or using primary, secondary and/or tertiary monoamines.

8. A binder as claimed in Claims 1 to 7, characterized in that the monofunctional components optionally used are used in quantities of no more than 95 mole percent, preferably no more than 50 mole percent and, more especially, in quantities of no more than 10 mole percent, based on the mixture of the monofunctional components and the dialcohols or dicarboxylic acids, in the production of the polyester oligomers.

9. A binder as claimed in Claims 1 to 8, characterized in that the resorbable (meth)acrylate components are spreadable as liquids or pastes at room temperature and preferably have a viscosity at room temperature of from about 500 to 70,000 mPa.s.

10. A binder as claimed in Claims 1 to 9, characterized in that it contains as starter organoboron compounds which are substantially non-inflammable in air, but which are capable of initiating polymerization in air at body temperature.

11. A binder as claimed in Claims 1 to 10, characterized in that it contains as starter an organoboron compound which contains alkyl and/or aryl residues and which may also contain B-H-bonds.

12. A binder as claimed in Claims 1 to 11, characterized in that it contains as starter at least one boron trialkyl compound and/or at least one boron alkyl hydride compound, in this case in particular a dialkyl borohydride.

13. A binder as claimed in Claims 1 to 12, characterized in that it is present as a two-component system of which the components - polymerizable component (A) and starter — containing component (B) - are mixed together for use or are even processed by the no-mix process.

14. A binder as claimed in Claims 1 to 13, characterized in that the starter component (B) present consists of at least one of the following systems:

a) a homogeneous mixture of at least one air-activatable organoboron compound with at least one organic oligomer or polymer which is liquid to solid at room temperature, preferably compatible with component (A) and inert to the organoboron compounds;

b) polymeric organoboron compounds containing as substituents boron hydride and/or organoboron residues on an air-stable polymer matrix;

c) boron alkyl compounds containing boron hydride residues and/or organoboron residues on fatty acid and/or fatty alcohol esters.

15. A binder as claimed in Claim 14, characterized in that the oligomer or polymer according to a) is a polymer based on derivatives - preferably esters and/or optionally substituted acid amides - of (meth)acrylic acid which has been produced in situ, particularly in the absence of oxygen,from its monomers under the influence of the organoboron compounds.

16. The use of the binder systems claimed in Claims 1 to 15 for the production of resorbable moldings in the bonding of hard body tissue, optionally together with plastics and/or metal.

**Claims** for the Contracting State AT

1. A process for producing a surgical binder system suitable for bonding hard body tissue based on a polymerizable (meth)acrylate adhesive and a polymerization— initiating starter, characterized in that it contains (meth)acrylates - liquid to solid at room temperature with (meth)acrylate residues on polyester-oligomer chains of hydroxycarboxylic acids as the resorbable (meth)acrylate component and an organoboron compound as starter.

2. A process as claimed in Claim 1, characterized in that the resorbable (meth)acrylate component contains at least proportionally more than one (meth)acrylate residue, preferably two (meth)acrylate residues, on the

hydroxycarboxylic acid polyester oligomers.

3. A process as claimed in Claims 1 and 2, characterized in that the (meth)acrylate residues are at least proportionally present at the ends of the polyester oligomer chains, preferably two (meth)acrylate residues being proprovided in the α,ω-position.

4. A process as claimed in Claims 1 to 3, characterized in that the polyester oligomers are formed from monohydroxy monocarboxylic acids containing preferably 2 to 10 and, more particularly, 2 to 6 carbon atoms.

5. A process as claimed in Claims 1 to 4, characterized in that the polyester oligomers are synthesized from glycolic acid, lactic acid, hydroxypropionic acid, hydroxybutyric acid and/or hydroxybenzoic acid.

6. A process as claimed in Claims 1 to 5, characterized in that the polyester oligomers have an average molecular weight of from about 200 to 600 and preferably of from about 300 to 500.

7. A process as claimed in Claims 1 to 6, characterized in that the polyester oligomers have been produced using monofunctional and/or preferably difunctional alcohols or carboxylic acids or carboxylic acid anhydrides and/or using primary, secondary and/or tertiary monoamines.

8. A binder as claimed in Claims 1 to 7, characterized in that the monofunctional components optionally used are used in quantities of no more than 95 mole percent, preferably no more than 50 mole percent and, more especially, in quantities of no more than 10 mole percent, based on the mixture of the monofunctional components and the dialcohols or dicarboxylic acids, in the production of the polyester oligomers.

9. A process as claimed in Claims 1 to 8, characterized in that the resorbable (meth)acrylate components are spreadable as liquids or pastes at room temperature and preferably have a viscosity at room temperature of from about 500 to 70,000 mPa.s.

10. A process as claimed in Claims 1 to 9, characterized in that the starter used is an organoboron compound which is substantially non-inflammable in air, but which is capable of initiating polymerization in air at body temperature.

11. A process as claimed in Claims 1 to 10, characterized in that the starter used is an organoboron compound which contains alkyl and/or aryl residues and which may also contain B-H-bonds.

12. A process as claimed in Claims 1 to 11, characterized in that the starter used is at least one boron trialkyl compound and/or at least one boron alkyl hydride compound, in this case in particular a dialkyl borohydride.

13. A process as claimed in Claims 1 to 12, characterized in that a two-component system is produced of which the components - polymerizable component (A) and startercontaining component (B) - are mixed together for use or are even processed by the no-mix process.

14. A process as claimed in Claims 1 to 13, characterized in that the starter component (B) used consists of at least one of the following systems:

a) a homogeneous mixture of at least one air-activatable organoboron compound with at least one organic oligomer or polymer which is liquid to solid at room temperature, preferably compatible with component (A) and inert to the organoboron compounds;

b) polymeric organoboron compounds containing as substituents boron hydride and/or organoboron residues on an air-stable polymer matrix;

c) boron alkyl compounds containing boron hydride residues and/or organoboron residues on fatty acid and/or fatty alcohol esters.

15. A process as claimed in Claim 14, characterized in that the oligomer or polymer according to a) used is a polymer based on derivatives - preferably esters and/or optionally substituted acid amides - of (meth)acrylic acid which has been produced in situ, particularly in the absence of oxygen, from its monomers under the influence of the organoboron compounds.

16. The use of the binder systems claimed in Claims 1 to 15 for the production in situ of resorbable moldings in the bonding of hard body tissue, optionally together with plastics and/or metal.

**Revendications** pour les Etats Contractants BE CH DE FR GB IT LI NL SE

1. Système d'agents liants chirurgical approprié pour coller des tissus durs propres au corps, à base d'un adhésif de (méth)acrylate polymérisable et d'un initiateur déclenchant la polymérisation, caractérisé en ce que, comme composant (méth)acrylate résorbable, il contient des esters d'acide (méth)acrylique liquides à solides à la temperature ambiante et comportant des radicaux (méth)acrylate sur des chaînes oligomères de polyesters constituées d'acides hydroxycarboxyliques et, comme initiateur, des composés organiques de bore.

2. Agents liants selon la revendication 1, caractérisés en ce que le composant (méth)acrylate résorbable comporte au moins partiellement plus d'un radical (méth) acrylate, de préférence, deux radicaux (méth)acrylate, sur les oligomères de polyesters d'acides hydroxycarboxyliques.

3. Agents liants selon les revendications 1 et 2, caractérisés en ce que les radicaux (méth)acrylate se présentent au moins partiellement en terminaison sur les oligomères de polyesters en prévoyant, de préférence, deux radicaux (méth)acrylate en position α, ω.

4. Agents liants selon les revendications 1 à 3, caractérisés en ce que les oligomères de polyesters sont formés à partir d'acides monohydroxy— monocarboxyliques contenant, de préférence, 2 à 10, en particulier, 2 à 6 atomes de carbone.

5. Agents liants selon les revendications 1 à 4, caractérisés en ce que les oligomères de polyesters sont formés

à partir d'acide glycolique, d'acide lactique, d'acide hydroxypropionique, d'acide hydroxybutyrique et/ou d'acide hydroxybenzoïque.

6. Agents liants selon les revendications 1 à 5, caractérisés en ce que les oligoméres de polyesters ont un poids moléculaire moyen se situant dans l'intervalle allant d'environ 200 à 600, de preférence, dans l'intervalle allant d'environ 300 à 500.

7. Agents liants selon les revendications 1 à 6, caractérisés en ce qu'on prépare les oligomeres de polyesters en utilisant conjointement des anhydrides d'acides carboxyliques, des acides carboxyliques ou des alcools monofonctionnels et/ou, de preférence, difonctionnels, et/ou en utilisant conjointement des mono-amines primaires, secondaires et/ou tertiaires.

8. Agents liants selon les revendications 1 à 7, caractérisés en ce que, lors de la préparation des oligoméres de polyesters, on utilise les composants monofonctionnels éventuellement employés en quantités ne dépassant pas 95% molaires, de préférence, en quantités ne dépassant pas 50% molaires et, en particulier, en quantités ne dépassant pas 10% molaires, calculé chaque fois sur le mélange des composants monofonctionnels et des dialcools ou des acides dicarboxyliques.

9. Agents liants selon les revendications 1 à 8, caractérisés en ce que les composants (méth)acrylate résorbables sont aptes à l'enduction en un état se situant entre l'état fluide et l'état pâteux à la température ambiante et, en l'occurrence, ils ont, de préférence, à la température ambiante, une viscosité se situant dans l'intervalle allant d' environ 500 à 70.000 mPa.s.

10. Agents liants selon les revendications 1 à 9, caractérisés en ce que, comme initiateurs, ils contiennent des composés organiques de bore qui n'ont pratiquement aucune auto-inflammabilité à l'air, mais qui, en meme temps, sont aptes à déclencher une polymérisation à la température du corps avec apport d'air.

11. Agents liants selon les revendications 1 à 10, caractérisés en ce que, comme initiateurs, ils contiennent des composés organiques de bore comportant des radicaux alkyle et/ou aryle et pouvant également avoir des liaisons B-H.

12. Agents liants selon les revendications 1 à 11, caractérisés en ce que, comme initiateurs, ils contiennent au moins un composé de bore-trialkyle et/ou au moins un composé d'hydrure de bore-alkyle (dans ce cas, en particulier, un borohydrure de dialkyle).

13. Agents liants selon les revendications 1 à 12, caractérisés en ce qu'ils sont présents sous forme de systemes à deux composants dont les composants (composant polymérisable (A) et composant (B) contenant l'initiateur) sont mélangés l'un avec l'autre lors de l'application ou sont également traités selon le procédé "sans mélange".

14. Agents liants selon les revendications 1 à 13, caractérisés en ce que, comme composant initiateur (B), il y a au moins un des systèmes suivants:

a) un mélange homogène d'au moins un composé organique de bore pouvant être activé par apport d'air avec au moins un polymere ou un oligomère organique liquide à solide à la température ambiante, de préférence, compatible avec le composant (A) et inerte vis-à-vis des composés organiques de bore;

b) des composés organiques polymères de bore qui, comme substituants sur une matrice polymere stable vis-à-vis de l'apport d'air, comportent des radicaux d' hydrogène boré et/ou des radicaux organiques de bore;

c) des composés de bore-alkyle qui contiennent les radicaux d'hydrogéne boré et/ou les radicaux organiques de bore sur des esters d'acides gras et/ou d,alcools gras.

15. Agents liants selon la revendication 14, caractérisés en ce que l'oligomère ou le polymère selon a) est un polymère à base de dérivés (de préférence, des esters et/ou des amides d'acides éventuellement substitués) de l'acide (méth)acrylique que l'on prépare in situ à partir de ses monomères, en particulier, en absence d'oxygéne et sous l'influence des composés organiques de bore.

16. Utilisation des systèmes d'agents liants selon les revendications 1 à 15 pour rèaliser des pieces moulées résorbables lors du collage de tissus durs propres au corps éventuellement avec une matière synthétique et/ou un métal.

**Revendications** pour l'Etat Contractant AT

1. Procédé de préparation d'un système d'agents liants chirurgical approprié pour coller des tissus durs propres au corps, à base d'un adhésif de (méth)acrylate polymérisable et d'un initiateur déclenchant la polymérisation, caractérisé en ce que, comme composant (méth)acrylate résorbable, on utilise des esters d'acide (méth)acrylique liquides à solides à la température ambiante et comportant des radicaux (méth)acrylate sur des chaînes oligomères de polyesters formées à partir d' acides hydroxycarboxyliques et, comme initiateur, des composés organiques de bore.

2. Procédé selon la revendication 1, caractérisé en ce que le composant (méth)acrylate resorbable comporte au moins partiellement plus d'un radical (méth)acrylate, de préférence, deux radicaux (méth)acrylate, sur les oligomères de polyesters d'acides hydroxycarboxyliques.

3. Procédé selon les revendications 1 et 2, caractérisé en ce que les radicaux (méth)acrylate se présentent au moins partiellement en terminaison sur les oligomères de polyesters, deux radicaux (méth)acrylate étant, de préférence, prévus en position $\alpha$, $\omega$.

4. Procédé selon les revendications 1 à 3, caractérisé en ce que les oligomères de polyesters sont formés à

partir d'acides monohydroxy-monocarboxyliques contenant, de préférence, 2 à 10, en particulier, 2 à 6 atomes de carbone.

5. Procédé selon les revendications 1 à 4, caractérisé en ce que les oligomères de polyesters sont formés à partir de l'acide glycolique, de l'acide lactique, de l'acide hydroxypropionique, de l'acide hydroxybutyrique et/ou de l'acide hydroxybenzoïque.

6. Procédé selon les revendications 1 à 5, caractérisé en ce que les oligomères de polyesters ont un poids moléculaire moyen se situant dans l'intervalle allant d'environ 200 à 600, de préférence, dans l'intervalle allant d'environ 300 à 500.

7. Procédé selon les revendications 1 à 6, caractérisé en ce qu'on prépare les oligomères de polyesters en utilisant conjointement des anhydrides d'acides carboxyliques, des acides carboxyliques ou des alcools monofonctionnels et/ou, de préférence, difonctionnels, et/ou en utilisant conjointement des mono-amines primaires, secondaires et/ou tertiaires.

8. Procédé selon les revendications 1 à 7, caractérisé en ce que, lors de la préparation des oligomères de polyesters, on utilise les composants monofonctionnels éventuellement employés en quantités ne dépassant pas 95% molaires, de préférence, en quantités ne dépassant pas 50% molaires et, en particulier, en quantités ne dépassant pas 10% molaires, calculé chaque fois sur le mélange des composants monofonctionnels et des dialcools ou des acides dicarboxyliques.

9. Procédé selon les revendications 1 à 8, caractérisé en ce que les composants (méth)acrylate résorbables utilisés sont aptes à l'enduction en un état se situant entre l'état fluide et l'état pûteux à la température ambiante et, en l'occurrence, ils ont, de préférence, à la température ambiante, une viscosité se situant dans l'intervalle allant d'environ 500 à 70.000 mPa.s.

10. Procédé selon les revendications 1 à 9, caractérisé en ce que, comme initiateur, on utilise des composés organiques de bore qui n'ont pratiquement aucune auto-inflammabilité à l'air mais qui, en même temps, sont aptes à déclencher une polymérisation à la température du corps et avec apport d'air.

11. Procédé selon les revendications 1 à 10, caractérisé en ce que, comme initiateur, on utilise des composés organiques de bore comportant des radicaux alkyle et/ou aryle et pouvant également contenir des liaisons B-H.

12. Procédé selon les revendications 1 à 11, caractérisé en ce que, comme initiateur, on utilise au moins un composé de bore-trialkyle et/ou au moins un composé d'hydrure de bore-alkyle (dans ce cas, en particulier, un borohydrure de dialkyle).

13. Procédé selon les revendications 1 à 12, caractérisé en ce qu'on prépare un système à deux composants dont on mélange les composants (composant polymérisable (A) et composant (B) contenant l'initiateur) l'un avec l'autre lors de l'application, ces composants pouvant également être traités selon le procédé "sans mélange".

14. Procédé selon les revendications 1 ü 13, caractérisé en ce que, comme composant initiateur (B), on utilise au moins un des systèmes suivants:

a) un mélange homogéne d' au moins un composé organique de bore pouvant être activé par apport d'air avec au moins un polymère ou un oligomère organique liquide à solide à la température ambiante, de préférence, compatible avec le composant (A) et inerte vis-à-vis des composés organiques de bore;

b) des composés organiques polymères de bore qui, comme substituants sur une matrice polymère stable vis-à-vis de l'apport d'air, comportent des radicaux d' hydrogène boré et/ou des radicaux organiques de bore;

c) des composés de bore-alkyle contenant des radicaux d'hydrogène boré et/ou des radicaux organiques de bore sur des esters d'acides gras et/ou d'alcools gras.

15. Procédé selon la revendication 14, caractérisé en ce que, comme oligomère ou polymère selon a), on utilise un polymère à base de dérivés (de préférence, des esters et/ou des amides d'acides éventuellement substitués) de l'acide (méth) acrylique que l'on prépare in situ à partir de ses monomères, en particulier, en absence d'oxygène et sous l'influence des composés organiques de bore.

16. Utilisation des systèmes d'agents liants selon les revendications 1 à 15 pour la réalisation in situ de pièces moulées résorbables lors du collage de tissus durs propres au corps éventuellement avec une matière synthétique et/ou un métal.